# EUROPEAN PATENT APPLICATION

(11) **EP 4 134 205 A1**
(43) Date of publication of application: **15.02.2023**
(21) Application number: 21785658.2
(22) Date of filing: 07.04.2021
(51) Int. Cl.: B25J 3/00, G01N 35/04, A61B 34/20, A61B 34/35, A61B 34/37, A61B 50/30, A61B 90/20, A61G 12/00, G01N 1/10, B63B 35/44, A61B 5/00

(54) **MEDICAL MOVABLE BODY SYSTEM AND METHOD FOR DRIVING SAME**

(30) Priority: 10.04.2020 JP 2020071339; 29.05.2020 JP 2020094590
(71) Applicant: KAWASAKI JUKOGYO KABUSHIKI KAISHA, Kobe-shi, Hyogo 650-8670 (JP)
(72) Inventor: HASHIMOTO, Yasuhiko, Hyogo 650-8670 (JP); KAMEYAMA, Atsushi, Hyogo 650-8670 (JP); KAMON, Masayuki, Hyogo 650-8670 (JP)
(74) Representative: Müller-Boré & Partner Patentanwälte PartG mbB
(86) International application number: PCT/JP2021/014800
(87) International publication number: WO 2021/206124

(57) **Abstract**

A medical movable body system according to the present disclosure includes a medical movable body (200), a robot (101), a manipulator (102), and a controller (110). The robot (101) is in a first space (201). The manipulator (102) is in a second space (202). The controller (110) executes: (A) making the robot (101) self-travel to approach a patient; and after the (A), (B) operating an arm (13) and/or a hand (18).

## Description

### Technical Field

The present disclosure relates to a medical movable body system and a method of operating the medical movable body system.

### Background Art

Known is a self-traveling tray conveying robot which manages whether or not a patient is in a room and self-travels to supply to a blood collection base a blood collecting vessel storage tray of the patient who is in the room, the blood collecting vessel storage tray being prepared in a blood collecting vessel preparation room (see PTL 1, for example).

According to the self-traveling tray conveying robot disclosed in PTL 1, a noncontact medium such as RF-ID is used as an inspection reception slip, and a reader for the noncontact medium is at an entrance gate of a blood collection room. With this, entering and leaving of the patient into and from the room are checked.

Then, when the patient is not in the room, the self-traveling tray conveying robot receives the tray automatically prepared by the blood collecting vessel preparation device and stocks the tray in a predetermined stock portion. When the patient is in the blood collection room, the self-traveling tray conveying robot self-travels to supply the tray to the requested blood collection base. Thus, medical practices are assisted.

### Citation List

### Patent Literature

PTL 1: Japanese Laid-Open Patent Application Publication No. 2007-130282

### Summary of Invention

### Technical Problem

In recent years, infectious diseases by coronavirus, such as SARS, MERS, and COVID-19, are spreading. Moreover, infectious diseases by virus other than coronavirus and by various bacteria are also well-known.

For patients who are suspected to be infected with such infectious diseases, a specimen of a mucous membrane or the like is collected, and an inspection such as a PCR test is performed. Thus, whether or not the patient is infected is diagnosed. However, there is a possibility that a medical worker who collects the specimen is infected with virus or the like.

Moreover, there is a possibility that since the medical worker who has contacted the patient who was infected with virus or the like moves in a medical movable body, such as a hospital ship, in a period from when the inspection is performed until when the infection is confirmed, other patients and the like in the movable body are infected with the virus or the like.

The present inventors have found that a robot is operated by remote control to perform inspection practices and examination practices of patients who are suspected to be infected with virus or the like, and with this, the infection of medical workers and the like can be adequately reduced. Thus, the present disclosure was made.

An object of the present disclosure is to provide a medical movable body system and a method of operating the medical movable body system, each of which can adequately reduce the infection of medical workers and the like.

### Solution to Problem

To solve the above conventional problems, a medical movable body system according to the present disclosure is a medical movable body system including a medical movable body that accommodates patients of various diseases. The medical movable body system includes: the medical movable body; a robot including an arm including a hand that holds a medical inspection tool and/or a medical examination tool; a manipulator that manipulates the robot; and a controller. The robot is in a first space in the medical movable body. The manipulator is in a second space that is isolated from the first space. The controller executes: (A) making the robot self-travel to a vicinity of the patient; after the (A), (A) making the robot self-travel to approach the patient; and after the (A), (B) operating the arm and/or the hand based on manipulation command information of the arm and/or manipulation command information of the hand which are input from the manipulator.

With this, since the medical worker in the space isolated from the patient manipulates the robot, the contact with the patient who is suspected to be infected with virus or the like can be avoided. Therefore, the infection of the medical worker with virus or the like can be adequately avoided.

Moreover, since the robot moves to the vicinity of the patient in each hospitalization room or the like, the medical worker does not have to move in the medical movable body. Therefore, the infection of the medical worker can be adequately avoided.

Furthermore, since the robot for the patient who is suspected to be infected with an infectious disease and the robot for a patient who has another disease are separately used, the infection of the patient who has the another disease can be avoided.

Moreover, a medical movable body system according to the present disclosure is a medical movable body system including a medical movable body that accommodates patients of various diseases. The medical movable body system includes: the medical movable body; a robot including an arm including a hand that holds a medical inspection tool and/or a medical examination tool; a manipulator that manipulates the robot; and a controller. The robot is in a first space in the medical movable body. The manipulator is in a second space that is isolated from the first space. A first imager is at the hand. The controller executes (α) operating the arm and/or the hand based on manipulation command information of the arm and/or manipulation command information of the hand which are input from the manipulator.

With this, since the medical worker in the space isolated from the patient manipulates the robot, the contact with the patient who is suspected to be infected with virus or the like can be avoided. Therefore, the infection of the medical worker with virus or the like can be adequately avoided.

Moreover, a method of operating a medical movable body system according to the present disclosure is a method of operating a medical movable body system including a medical movable body that accommodates patients of various diseases. The medical movable body system includes: the medical movable body; a robot including an arm including a hand that holds a medical inspection tool and/or a medical examination tool; and a manipulator that manipulates the robot. The robot is in a first space in the medical movable body. The manipulator is in a second space that is isolated from the first space. The method includes: (A) making the robot self-travel to approach the patient; and after the (A), (B) operating the arm and/or the hand based on manipulation command information of the arm and/or manipulation command information of the hand which are input from the manipulator.

With this, since the medical worker in the space isolated from the patient manipulates the robot, the contact with the patient who is suspected to be infected with virus or the like can be avoided. Therefore, the infection of the medical worker with virus or the like can be adequately avoided.

Moreover, since the robot moves to the vicinity of the patient in each hospitalization room or the like, the medical worker does not have to move in the medical movable body. Therefore, the infection of the medical worker can be adequately avoided.

Furthermore, since the robot for the patient who is suspected to be infected with an infectious disease and the robot for a patient who has another disease are separately used, the infection of the patient who has the another disease can be avoided.

Moreover, a medical movable body system according to the present disclosure is a method of operating a medical movable body system including a medical movable body that accommodates patients of various diseases. The medical movable body system includes the medical movable body. The robot is in a first space in the medical movable body. The manipulator is in a second space that is isolated from the first space. A first imager is at the hand. The method includes (α) operating the arm and/or the hand based on manipulation command information of the arm and/or manipulation command information of the hand which are input from the manipulator.

With this, since the medical worker in the space isolated from the patient manipulates the robot, the contact with the patient who is suspected to be infected with virus or the like can be avoided. Therefore, the infection of the medical worker with virus or the like can be adequately avoided.

The above object, other objects, features, and advantages of the present disclosure will be made clear by the following detailed explanation of preferred embodiments with reference to the attached drawings.

### Advantageous Effects of Invention

According to the medical movable body system of the present disclosure and the method of operating the medical movable body system of the present disclosure, the infection of medical workers and the like in a medical movable body can be adequately reduced.

### Brief Description of Drawings

FIG. 1 is a schematic diagram showing a schematic configuration of a medical movable body system according to Embodiment 1.
FIG. 2 is a schematic diagram showing a schematic configuration of the medical movable body system according to Embodiment 1.
FIG. 3 is a schematic diagram showing a schematic configuration of a robot of the medical movable body system shown in FIGS. 1 and 2.
FIG. 4 is a schematic diagram showing a schematic configuration of a hand of the robot of the medical movable body system according to Embodiment 1.
FIG. 5 is a flowchart showing one example of operations of the medical movable body system according to Embodiment 1.
FIG. 6 is a schematic diagram showing one example of operations of the robot of the medical movable body system according to Embodiment 1.
FIG. 7 is a schematic diagram showing one example of image information and/or video information on a first display shown in FIG. 1.
FIG. 8 is a schematic diagram showing another example of the image information and/or the video information on the first display shown in FIG. 1.
FIG. 9 is a schematic diagram showing yet another example of the image information and/or the video information on the first display shown in FIG. 1.
FIG. 10 is a schematic diagram showing still another example of the image information and/or the video information on the first display shown in FIG. 1.
FIG. 11 is a schematic diagram showing a schematic configuration of the medical movable body system according to Modified Example 1 of Embodiment 1.
FIG. 12 is a schematic diagram showing a schematic configuration of the medical movable body system according to Embodiment 2.
FIG. 13 is a schematic diagram showing a schematic configuration of the medical movable body system according to Embodiment 3.
FIG. 14 is a schematic diagram showing a schematic configuration of the medical movable body system according to Embodiment 4.
FIG. 15 is a flowchart showing one example of the operations of the medical movable body system according to Embodiment 4.
FIG. 16 is a schematic diagram showing a schematic configuration of the medical movable body system according to Modified Example 1 of Embodiment 4.
FIG. 17 is a schematic diagram showing a schematic configuration of the medical movable body system according to Embodiment 5.
FIG. 18 is a flowchart showing one example of the operations of the medical movable body system according to Embodiment 5.
FIG. 19 is a schematic diagram showing a schematic configuration of the medical movable body system according to Embodiment 6.
FIG. 20 is a schematic diagram showing a schematic configuration of the medical movable body system according to Embodiment 6.
FIG. 21 is a schematic diagram showing a schematic configuration of the hand of the robot shown in FIG. 19.
FIG. 22 is a flowchart showing one example of the operations of the medical movable body system according to Embodiment 6.
FIG. 23 is a schematic diagram showing a schematic configuration of the medical movable body system according to Modified Example 1 of Embodiment 6.
FIG. 24 is a schematic diagram showing a schematic configuration of the medical movable body system according to Embodiment 7.
FIG. 25 is a schematic diagram showing a schematic configuration of the medical movable body system according to Embodiment 8.
FIG. 26 is a flowchart showing one example of the operations of the medical movable body system according to Embodiment 8.

### Description of Embodiments

Hereinafter, embodiments of the present disclosure will be described with reference to the drawings. In the drawings, the same reference signs are used for the same or corresponding components, and the repetition of the same explanation is avoided. Moreover, in the drawings, components for describing the present disclosure are selectively shown, and the other components may be omitted. Furthermore, the present disclosure is not limited to the following embodiments.

### Embodiment 1

A medical movable body system according to Embodiment 1 is a medical movable body system including a medical movable body that accommodates patients of various diseases. The medical movable body system includes: the medical movable body; a robot including an arm including a hand that holds a medical inspection tool and/or a medical examination tool; a manipulator that manipulates the robot; and a controller. The robot is in a first space in the medical movable body. The manipulator is in a second space that is isolated from the first space. The controller executes: (A) making the robot self-travel to approach the patient; and after the (A), (B) operating the arm based on manipulation command information of the arm and/or manipulation command information of the hand which are input from the manipulator.

Moreover, the medical movable body system according to Embodiment 1 may further include: a first imager; and a first display that displays image information of an image taken by the first imager and/or video information of a video taken by the first imager. The controller may execute the (B) in a state where the first display displays the image information of the image taken by the first imager and/or the video information of the video taken by the first imager.

Moreover, in the medical movable body system according to Embodiment 1, a pair of laser beam indicators may be at the hand such that light beams emitted from the laser beam indicators intersect with each other.

Moreover, in the medical movable body system according to Embodiment 1, the first imager may be at the robot or the hand of the robot.

Moreover, in the medical movable body system according to Embodiment 1, the first display may display a virtual model showing positional information of the medical inspection tool and/or positional information of the medical examination tool.

Moreover, in the medical movable body system according to Embodiment 1, the manipulator may include a manipulation switch that instructs unholding of the medical inspection tool and/or the medical examination tool.

Moreover, in the medical movable body system according to Embodiment 1, the robot and the manipulator may operate in a master-slave mode.

Moreover, in the medical movable body system according to Embodiment 1, the second space may be located outside the medical movable body.

Moreover, in the medical movable body system according to Embodiment 1, the medical movable body may be a hospital ship.

A method of operating a medical movable body system according to Embodiment 1 is a method of operating a medical movable body system including a medical movable body that accommodates patients of various diseases. The medical movable body system includes: the medical movable body; a robot including an arm including a hand that holds a medical inspection tool and/or a medical examination tool; and a manipulator that manipulates the robot. The robot is in a first space in the medical movable body. The manipulator is in a second space that is isolated from the first space. The method includes: (A) automatically moving the robot to a vicinity of the patient based on positional information of the patient which is input from the manipulator; and after the (A), (B) operating the arm and/or the hand based on manipulation command information of the arm and/or manipulation command information of the hand which are input from the manipulator.

Moreover, in the method of operating the medical movable body system according to Embodiment 1, the medical movable body system may further include: a first imager; and a first display that displays image information of an image taken by the first imager and/or video information of a video taken by the first imager. The method may execute the (B) in a state where the first display displays the image information of the image taken by the first imager and/or the video information of the video taken by the first imager.

Moreover, in the method of operating the medical movable body system according to Embodiment 1, a pair of laser beam indicators may be at the hand such that light beams emitted from the laser beam indicators intersect with each other.

Moreover, in the method of operating the medical movable body system according to Embodiment 1, the first imager may be at the robot or the hand of the robot.

Moreover, in the method of operating the medical movable body system according to Embodiment 1, the first display may display a virtual model showing positional information of the medical inspection tool and/or positional information of the medical examination tool.

Moreover, in the method of operating the medical movable body system according to Embodiment 1, the first display may display a virtual model of a medical practice target part of the patient.

Moreover, in the method of operating the medical movable body system according to Embodiment 1, the manipulator may include a manipulation switch that instructs unholding of the medical inspection tool and/or the medical examination tool.

Moreover, in the method of operating the medical movable body system according to Embodiment 1, the robot and the manipulator may operate in a master-slave mode.

Moreover, in the method of operating the medical movable body system according to Embodiment 1, the second space may be located outside the medical movable body.

Moreover, in the method of operating the medical movable body system according to Embodiment 1, the medical movable body may be a hospital ship.

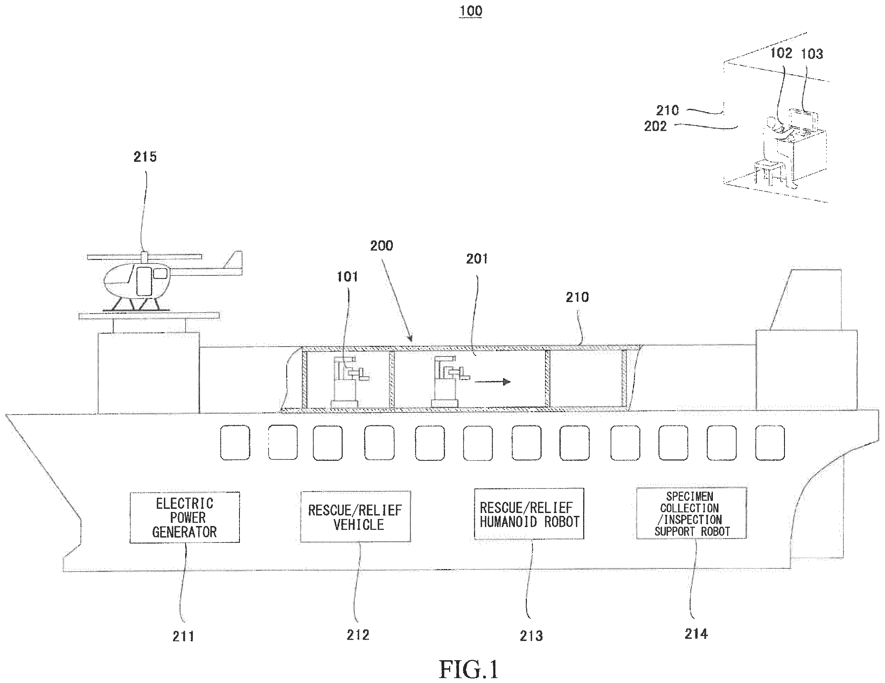

Hereinafter, one example of the medical movable body system according to Embodiment 1 will be described with reference to FIGS. 1 to 10.

### Configuration of Medical Movable Body System

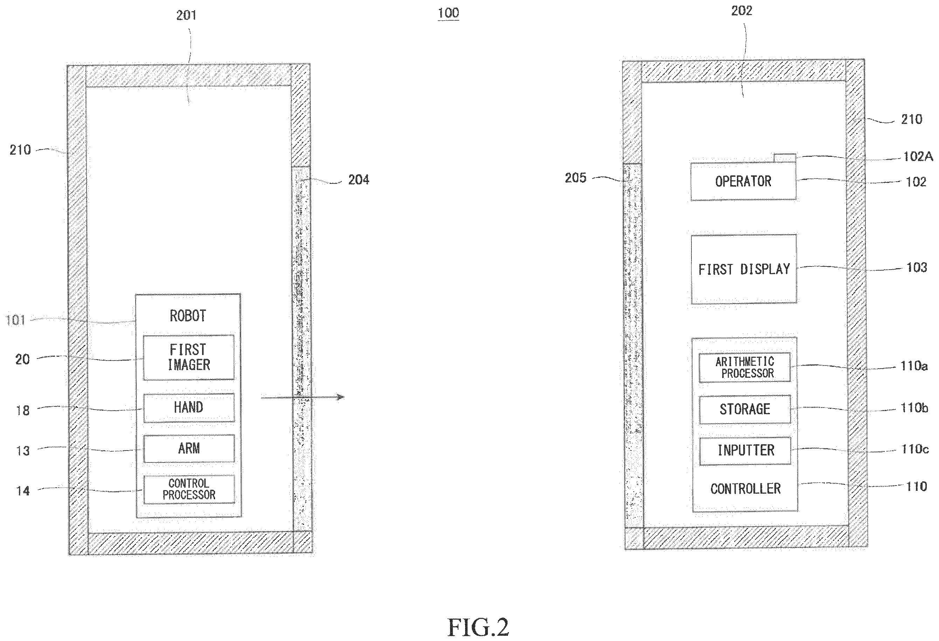

FIGS. 1 and 2 are schematic diagrams each showing a schematic configuration of the medical movable body system according to Embodiment 1.

As shown in FIG. 1, a medical movable body system 100 according to Embodiment 1 includes a medical movable body 200, a robot 101, a manipulator 102, a first display 103, and a controller 110. The robot 101 is at the medical movable body 200, and the manipulator 102 and the first display 103 are located so as to be isolated from the medical movable body 200.

Specifically, the robot 101 is in a first space 201 that is a space in the medical movable body 200. Moreover, the manipulator 102, the first display 103, and the controller 110 are in a second space 202 that is isolated from the first space 201.

The second space 202 may be inside the medical movable body 200 or may be outside the medical movable body 200 as long as the second space 202 is isolated from the first space 201. The outside of the medical movable body 200 may be a hospital.

Moreover, an imager (first imager) that takes an image of a lateral side of a face of the patient may be inside the first space 201. The imager may be on a dividing wall 210 that defines the first space 201 or may be gripped by a robot that is different from the robot 101.

Moreover, tools by which the robot 101 performs medical inspection practices, experimental equipment, various inspection reagents, and the like may be in a room (examination room/inspection room) that defines the first space 201. Examples of the tools include automatic pipettes, chips for automatic pipettes, microtubes, centrifuge tubes, and centrifugal settling tubes. Examples of the experimental equipment include centrifuges and PCR devices.

The medical movable body 200 can accommodate patients of various diseases and move. Examples of the medical movable body 200 include a hospital ship and a railcar. In the medical movable body 200, the first space 201 is defined by the dividing wall 210.

Moreover, various rescue/relief equipment, such as an electric power generator 211, a rescue/relief vehicle 212, a rescue/relief humanoid robot 213, a specimen collection/inspection support robot 214, and an emergency medical helicopter 215, may be at the medical movable body 200.

The rescue/relief humanoid robot 213 and/or the specimen collection/inspection support robot 214 may be the same in type as the robot 101 or may be different in type from the robot 101.

For example, the second space 202 may be defined inside the medical movable body 200 by the dividing wall 210. Moreover, the second space 202 may be defined inside a hospital, which is the outside of the medical movable body 200, by the dividing wall 210.

A front room may be at a room (control room) that defines the second space 202. Moreover, a fan filter structure may be in the front room. The fan filter structure sets pressure of the front room to negative pressure and sets pressure of the second space 202 (internal space of the control room) to positive pressure. A known fan filter structure may be used as the fan filter structure.

Moreover, the dividing wall 210 may include a shutter (door) 204 that allows or inhibits access to the first space 201 and may include a shutter (door) 205 that allows or inhibits access to the second space 202.

Moreover, a part of the dividing wall 210 may be a transparent body, such as a glass plate, and therefore, an operator (medical worker) and the like may see an inside of the first space 201.

The manipulator 102 manipulates the robot 101. A known manipulator, such as a joystick, a keyboard, a numeric keypad, or a teach pendant, may be used as the manipulator 102.

Moreover, equipment that transmits force information detected by a force sensor at a hand 18 of the below-described robot 101 or voice information to the operator may be at the manipulator 102. Examples of such equipment include a vibrating motor, a speaker, and a structure that expands or contracts a casing including a gripper.

The manipulator 102 may be carried by the operator (medical worker), i.e., may be portable. Moreover, the robot 101 and the manipulator 102 may operate in a master-slave mode.

Moreover, the manipulator 102 may include a release button 102A that releases a medical inspection tool or a medical examination tool, which is held by the hand 18, in emergency (for example, in a case where the robot 101 operates abnormally). When the release button 102A is pushed by the operator, the controller 110 may operate the robot 101 such that the hand 18 moves away from the patient.

The first display 103 displays image information of an image taken by a below-described first imager 20 and/or video information of a video taken by the first imager 20. The first display 103 may be a stationary display which is used by being placed on a desk, a floor, or the like. Moreover, the first display 103 may be a head mount display or a pair of eyeglasses which is used by being put on the operator.

The robot 101 can self-travel to the vicinity of the patient based on positional information of the patient which is input from the manipulator 102, positional information (such as positional information of a patient's room or an examination room) inside a medical movable body, and/or the like.

Moreover, the robot 101 operates the arm and/or the hand based on manipulation information of the arm and/or manipulation information of the hand which are input from the manipulator 102. At this time, the robot 101 may automatically move by the control of the controller 110 in accordance with work contents of a medical practice (for example, an examination and/or an inspection) to be executed to the patient such that a distance between the robot 101 and the patient is maintained at a predetermined first distance that is set in advance.

The following will describe an example in which a practice (examination) of listening to a front side of the patient with a stethoscope is performed, and then, a practice (examination) of listening to a back side of the patient with the stethoscope is performed. When the patient shows his/her back side to the robot 101, the robot 101 may automatically move to a rear side of the patient and then may automatically move so as to approach the patient (maintain the first distance).

With this, the medical worker can operate the robot 101 by remote control and can perform the medical practice (for example, the examination and/or the inspection) to the patient.

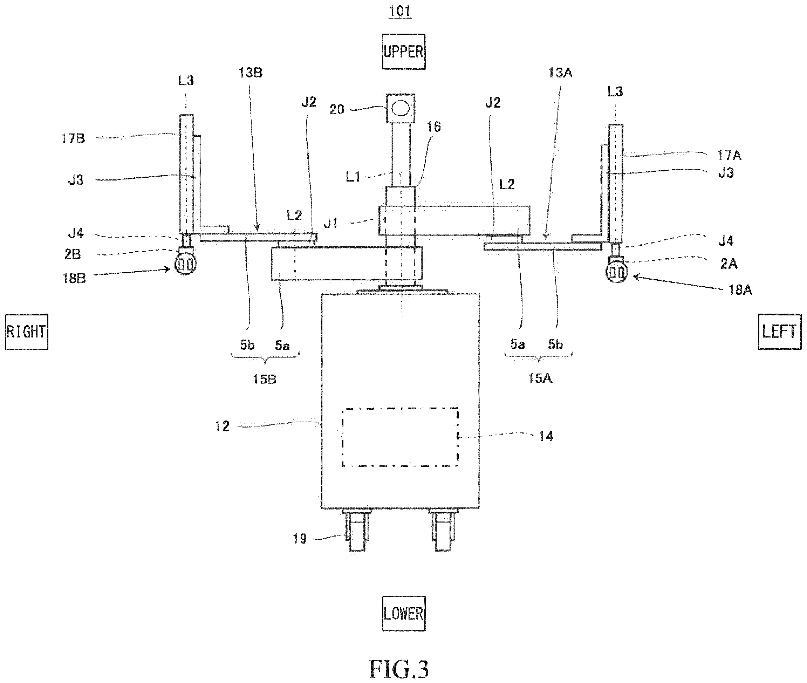

Herein, the configuration of the robot 101 will be described in detail with reference to FIG. 3. The following will describe a case where the robot 101 is a horizontal articulated double arm robot. However, another robot, such as a horizontal articulated robot or a vertical articulated robot, may be adopted as the robot 101.

FIG. 3 is a schematic diagram showing a schematic configuration of the robot of the medical movable body system shown in FIGS. 1 and 2. In FIG. 3, an upper-lower direction of the robot is shown as an upper-lower direction in the drawing.

As shown in FIG. 3, the robot 101 includes a cart 12, a first arm 13A, a second arm 13B, a first hand 18A, a second hand 18B, and a control processor 14 located in the cart 12.

When the first arm 13A and the second arm 13B are not distinguished from each other, the first arm 13A and the second arm 13B are simply called an "arm 13." Similarly, when the first hand 18A and the second hand 18B are not distinguished from each other, the first hand 18A and the second hand 18B are simply called a "hand 18."

Moreover, Embodiment 1 adopts a case where the control processor 14 is in the cart 12. However, the present embodiment is not limited to this, and the control processor 14 may be located outside the cart 12. The control processor 14 will be described later.

Wheels 19 are located on a lower surface of the cart 12. A suitable gear and a suitable drive motor are connected to the wheels 19. With this, the robot 101 can self-travel.

Moreover, a base shaft 16 and the first imager 20 are fixed to an upper surface of the cart 12. The first imager 20 takes an image and/or a video and outputs the image information of the taken image and/or the video information of the taken video to the controller 110. For example, the first imager 20 may be a video camera or an X-ray imager.

The first imager 20 may output the image information of the taken image and/or the video information of the taken video to the first display 103 without through the controller 110. The first imager 20 may be gripped by an arm other than the first arm 13A and the second arm 13B.

The first arm 13A and the second arm 13B are located at the base shaft 16 so as to be rotatable about a rotation axis L1 passing through a center axis of the base shaft 16. Specifically, the first arm 13A and the second arm 13B are located so as to be different in height from each other in the upper-lower direction. The first arm 13A and the second arm 13B can operate independently or in association with each other.

The first arm 13A includes a first arm portion 15A, a first wrist portion 17A, the first hand 18A, and a first attaching portion 2A. Similarly, the second arm 13B includes a second arm portion 15B, a second wrist portion 17B, the second hand 18B, and a second attaching portion 2B. Since the second arm 13B is the same in configuration as the first arm 13A, the detailed explanation thereof is omitted.

In Embodiment 1, the first arm portion 15A includes a first link 5a having a substantially rectangular solid shape and a second link 5b. A rotary joint J1 is at a base end portion of the first link 5a, and a rotary joint J2 is at a tip portion of the first link 5a. Moreover, a linear motion joint J3 is at a tip portion of the second link 5b.

Then, the base end portion of the first link 5a is coupled to the base shaft 16 through the rotary joint 11. The first link 5a is rotatable about the rotation axis L1 by the rotary joint J1. Moreover, a base end portion of the second link 5b is coupled to the tip portion of the first link 5a through the rotary joint J2. The second link 5b is rotatable about a rotation axis L2 by the rotary joint J2.

The first wrist portion 17A is coupled to the tip portion of the second link 5b through the linear motion joint J3 so as to be able to move up and down relative to the second link 5b. A rotary joint J4 is at a lower end portion of the first wrist portion 17A, and the first attaching portion 2A is at a lower end portion of the rotary joint J4.

The first hand 18A is attachable to and detachable from the first attaching portion 2A. Specifically, for example, the first attaching portion 2A includes a pair of rods which can adjust an interval therebetween. The first attaching portion 2A can sandwich the first hand 18A by the pair of rods and attach the first hand 18A to the first wrist portion 17A. With this, the first hand 18A can rotate about a rotation axis L3 by the rotary joint J4. A tip portion of each rod may be bent.

The first hand 18A may have any configuration as long as the first hand 18A holds the medical inspection tool or the medical examination tool. For example, as shown in FIGS. 1 and 3, the first hand 18A may hold the medical inspection tool or the medical examination tool by two claws. Examples of the medical inspection tool include: a sterilized swab; various tubes, such as a tube with a screw cap; a syringe; a catheter; and an endoscope inspection tool. Moreover, examples of the medical examination tool include a stethoscope and a tongue depressor.

Moreover, the first hand 18A can hold various workpieces, such as medicines, meals, and inspection reagents, and release such workpieces.

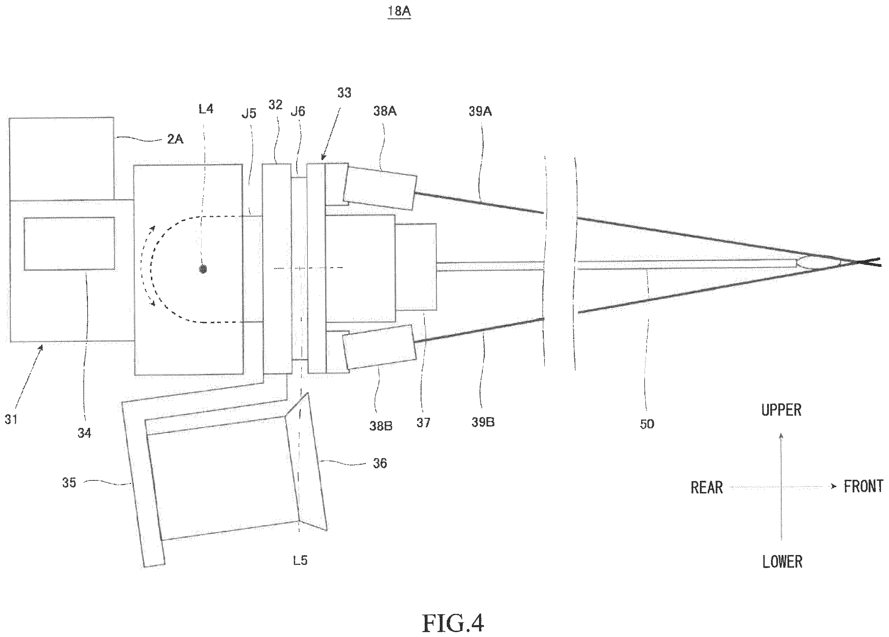

Herein, another example of the first hand 18A (hand 18) will be described with reference to FIG. 4.

FIG. 4 is a schematic diagram showing a schematic configuration of the hand of the robot in the medical movable body system according to Embodiment 1. In FIG. 4, the upper-lower direction and the front-rear direction of the robot are respectively shown as the upper-lower direction and the front-rear direction in the drawing.

As shown in FIG. 4, the first hand 18A includes a main body 31, an intermediate body 32, and a holder 33. The main body 31 and the intermediate body 32 are coupled to each other through a rotary joint J5. Moreover, the intermediate body 32 and the holder 33 are coupled to each other through a rotary joint J6. With this, the holder 33 is rotatable about a rotation axis L4 and/or a rotation axis L5 relative to the main body 31.

An actuator 34 that rotates the holder 33 is at the main body 31. For example, the actuator 34 may be a servomotor that is servo-controlled by the control processor 14. Moreover, a rotation sensor (not shown) and a current sensor (not shown) are at the main body 31. The rotation sensor detects a rotational position of the servomotor, and the current sensor detects a current by which the rotation of the servomotor is controlled. The rotation sensor may be, for example, an encoder. Positional information detected by the rotation sensor and current information detected by the current sensor may be output to the controller 110 through the control processor 14.

A support 35 is at a lower end portion of the intermediate body 32. A camera (first imager) 36 is attached to the support 35. The camera 36 takes an image and/or a video and outputs image information of the taken image and/or video information of the taken video to the controller 110. For example, the camera 36 may be a video camera or an X-ray imager.

Moreover, when collecting a nasopharynx swab fluid by the sterilized swab, a target whose image is taken by the camera 36 may be, for example, a nostril of the patient. Furthermore, for example, when collecting saliva or a specimen (sputum, etc.) derived from the lower airway by a suction catheter, the target whose image is taken by the camera 36 may be an oral cavity of the patient.

Embodiment 1 adopts a case where the support 35 and the camera 36 are at the lower end portion of the intermediate body 32. However, the present embodiment is not limited to this. The support 35 and the camera 36 may be at, for example, an upper end portion of the intermediate body 32. Moreover, the support 35 and the camera 36 may be at the holder 33.

A chuck structure 37 that holds or releases the medical inspection tool or the medical examination tool is attached to the holder 33. The chuck structure 37 may be, for example, an air chuck. Herein, the chuck structure 37 holds a sterilized swab 50 that collects a specimen for a PCR test.

Moreover, a pair of laser pointers (laser beam indicators) 38A and 38B are at the holder 33. The laser pointers 38A and 38B are located such that a laser beam 39A checked from the laser pointer 38A and a laser beam 39B emitted from the laser pointer 38B intersect with each other at a position in front of the first hand 18A. Three or more laser beam indicators may be at the first hand 18A.

With this, when the first hand 18A approaches the patient, a distance between the laser beam 39A and the laser beam 39B on the patient decreases. When the first hand 18A further approaches the patient, the laser beams on the patient become one spot. When the first hand 18A further approaches the patient, the distance between the laser beam 39A and the laser beam 39B on the patient increases.

Therefore, the operator (medical worker) can easily understand the distance between the patient and the tip portion of the medical inspection tool and/or the distance between the patient and the tip portion of the medical examination tool (the distance between the patient and the tip portion of the sterilized swab 50) based on the laser beams 39A and 39B emitted from the laser pointers 38A and 38B.

Moreover, at each of the joints J1 to J4 of the first arm 13A and the second arm 13B, a drive motor (not shown) is located as one example of an actuator that rotates two members, coupled to each other by the joint, relative to each other or moves the two members up and down relative to each other. The drive motor may be, for example, a servomotor that is servo-controlled by the control processor 14. Moreover, a rotation sensor (not shown) and a current sensor (not shown) are located at each of the joints J1 to J4. The rotation sensor detects a rotational position of the drive motor, and the current sensor detects a current by which the rotation of the drive motor is controlled. The rotation sensor may be, for example, an encoder. Positional information detected by the rotation sensor and current information detected by the current sensor may be output to the controller 110 through the control processor 14.

The control processor 14 includes an arithmetic processor and a storage (both not shown). The arithmetic processor is a microprocessor, a CPU or the like and controls various operations of the robot 101 by reading and executing software, such as a basic program, stored in the storage.

The storage stores information, such as the basic program and various fixed data. The storage may prestore, for example, map information of an inside of the medical movable body.

The storage does not have to be a single storage and may include storages (for example, a random access memory and a hard disc drive). When the arithmetic processor is a microcomputer, at least a part of the storage may be an internal memory of the microcomputer or may be an independent memory.

Moreover, the control processor 14 may control various operations of the robot 101 based on various command information input from the controller 110.

As shown in FIGS. 1 and 2, the controller 110 includes an arithmetic processor 110a, a storage 110b, and an inputter (manipulator) 110c. The arithmetic processor 110a is a microprocessor, a CPU, or the like and controls various operations of the medical movable body system 100 by reading and executing software, such as a basic program, stored in the storage 110b.

The storage 110b stores information, such as the basic program and various fixed data. The storage 110b does not have to be a single storage and may include storages (for example, a random access memory and a hard disc drive). When the arithmetic processor 110a is a microcomputer, at least a part of the storage 110b may be an internal memory of the microcomputer or may be an independent memory.

Various parameters regarding the control of the medical movable body system 100, the other data, and the like can be input to the arithmetic processor 110a through the inputter 110c. The inputter 110c is a known inputter, such as a keyboard, a touch panel, or a button switch group. In Embodiment 1, for example, the positional information of the patient may be able to be input through the inputter 110c. Moreover, the positional information of the patient may be able to be input though the manipulator 102.

The controller 110 may be a single controller 110 which performs centralized control or may include controllers 110 which cooperate to perform distributed control. Moreover, the controller 110 may be a microcomputer or may be a MPU, a PLC (Programmable Logic Controller), a logic circuit, or the like.

### Operations and Operational Advantages of Medical Movable Body System

Next, operations and operational advantages of the medical movable body system 100 according to Embodiment 1 will be described in detail with reference to FIGS. 1 to 10. The following operations are executed in such a manner that the arithmetic processor 110a of the controller 110 reads the program stored in the storage 110b.

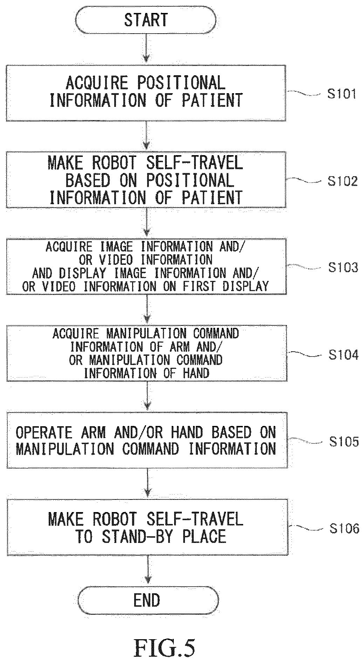

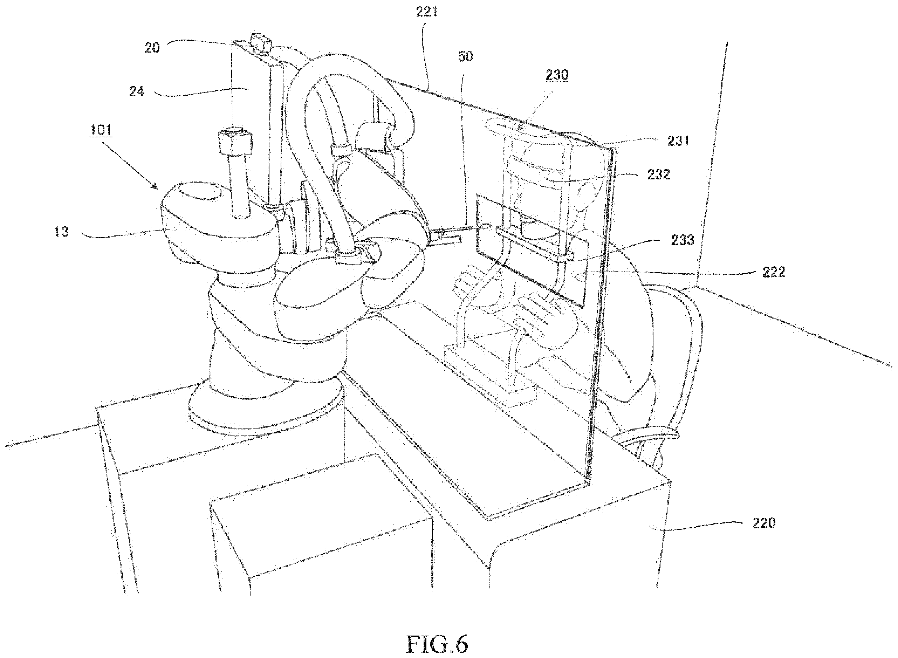

FIG. 5 is a flowchart showing one example of the operations of the medical movable body system according to Embodiment 1. FIG. 6 is a schematic diagram showing one example of the operations of the robot of the medical movable body system according to Embodiment 1.

As shown in FIG. 5, when the operator manipulates the inputter 110c (and/or the manipulator 102), the controller 110 acquires the positional information of the patient from the inputter 110c (and/or the manipulator 102) (Step S101).

Next, based on the positional information of the patient acquired in Step S101, the controller 110 makes the robot 101 self-travel (automatically move) from a preset stand-by place to the vicinity of the patient (Step S102).

Specifically, the controller 110 outputs to the control processor 14 the positional information of the patient which is acquired in Step S101. Based on the input positional information of the patient and the map information of the inside of the hospital which is stored in the storage, the control processor 14 drives the drive motor to make the robot 101 self-travel to the vicinity of the patient.

The stand-by place may be a room (space) that is isolated from the first space 201 and the second space 202.

Next, the controller 110 acquires the image information of the image taken by the first imager 20 and/or the video information of the video taken by the first imager 20 and displays the image information and/or the video information on the first display 103 (Step S103). The controller 110 may execute Step S103 before Step S101 or Step S102.

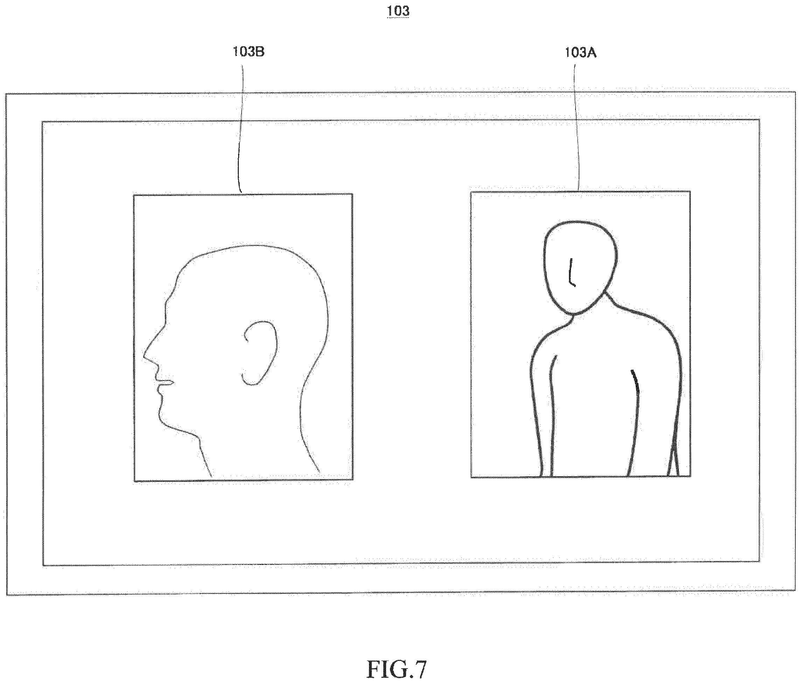

Herein, the image information and/or the video information displayed on the first display 103 will be described with reference to FIGS. 7 to 10.

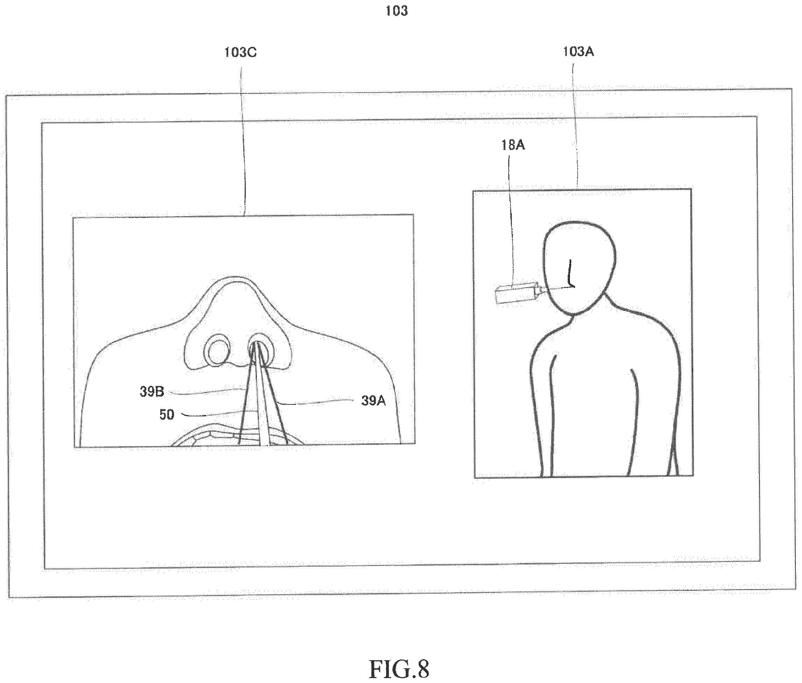

FIG. 7 is a schematic diagram showing one example of the image information and/or the video information displayed on the first display shown in FIG. 1. FIGS. 8 to 10 are schematic diagrams each showing another example of the image information and/or the video information displayed on the first display shown in FIG. 1. In FIGS. 8 to 10, a part of the robot 101 and a part of the first hand 18A are not shown.

As shown in FIG. 7, the video information (video information of the front side of the patient) of the video taken by the first imager 20 may be displayed as first video information 103A on the first display 103. Moreover, the video information of a video taken by an imager (not shown) that takes a video of the lateral side of the face of the patient may be displayed as second video information 103B on the first display 103.

As shown in FIG. 8, the video information of the video taken by the first imager 20 may be displayed as the first video information 103A on the first display 103. Moreover, the video information of the video taken by the camera 36 located at the first hand 18A may be displayed as third video information 103C on the first display 103.

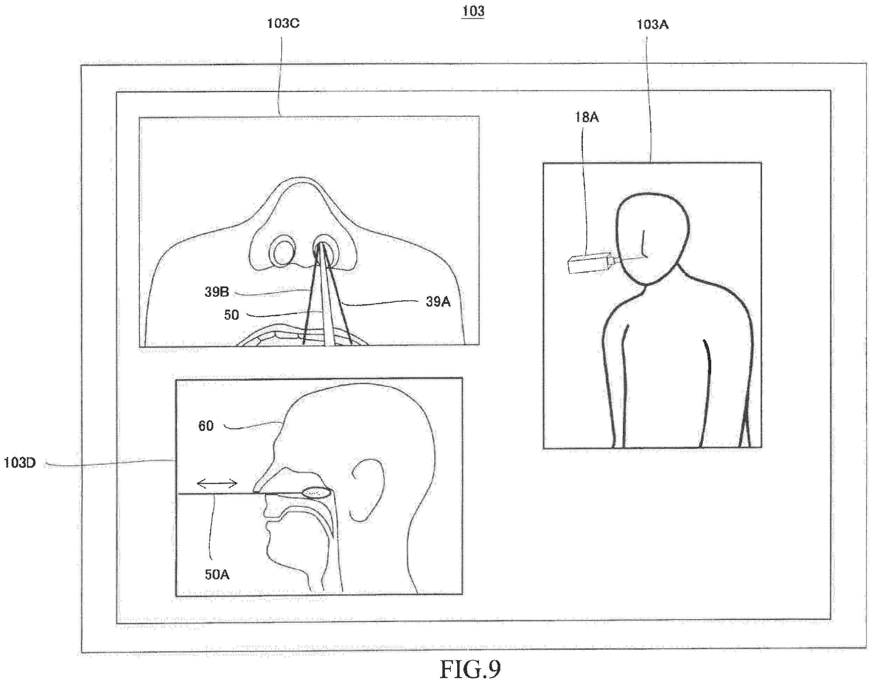

As shown in FIG. 9, the video information of the video taken by the first imager 20 may be displayed as the first video information 103A on the first display 103. Moreover, the video information of the video taken by the camera 36 located at the first hand 18A may be displayed as the third video information 103C on the first display 103.

Moreover, a virtual model showing the positional information of the medical inspection tool and/or the positional information of the medical examination tool may be displayed as fourth video information 103D on the first display 103. Specifically, a virtual sterilized swab 50A and a virtual patient 60 are displayed as the fourth video information 103D. The virtual sterilized swab 50A is a virtual model of the sterilized swab 50, and the virtual patient 60 is a virtual model of a medical target part of the patient.

At this time, the controller 110 may move the virtual sterilized swab 50A in the fourth video information 103D based on the positional information of the patient, the positional information detected by the rotation sensors that detect the rotational positions of the drive motors, and/or the manipulation information input to the manipulator 102. With this, the operator can easily understand the distance between the patient and the tip portion of the medical inspection tool and/or the distance between the patient and the tip portion of the medical examination tool (the distance between the patient and the tip portion of the sterilized swab 50).

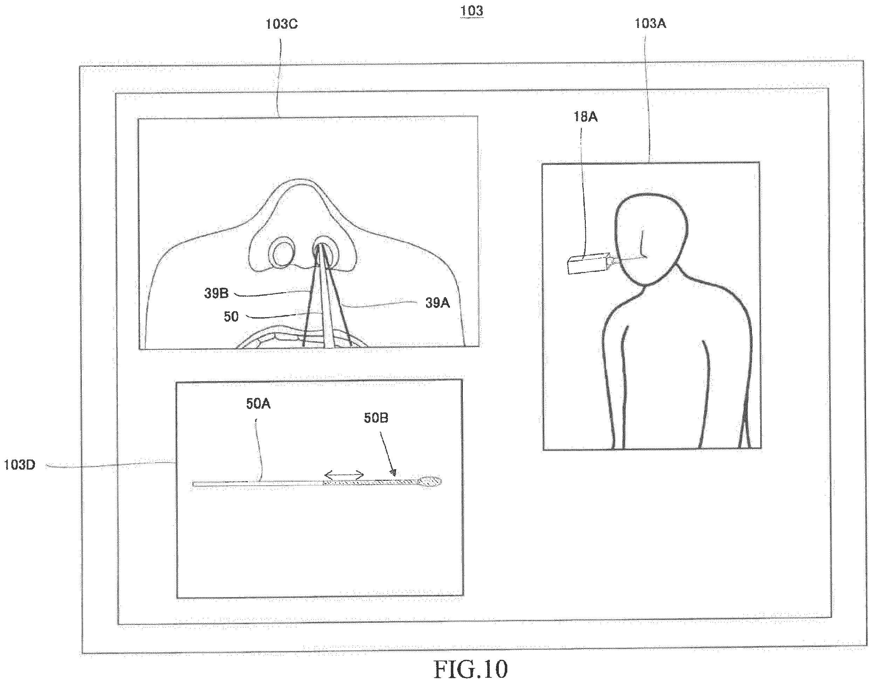

As shown in FIG. 10, the video information of the video taken by the first imager 20 may be displayed as the first video information 103A on the first display 103. Moreover, the video information of the video taken by the camera 36 located at the first hand 18A may be displayed as the third video information 103C on the first display 103.

Moreover, a virtual model showing the positional information of the medical inspection tool and/or the positional information of the medical examination tool may be displayed as the fourth video information 103D on the first display 103. Specifically, the virtual sterilized swab 50A that is a virtual model of the sterilized swab 50 is displayed as the fourth video information 103D.

At this time, based on the positional information of the patient, the positional information detected by the rotation sensors that detect the rotational positions of the drive motors, and/or the manipulation information input to the manipulator 102, the controller 110 may display a region of the sterilized swab 50, which has entered into the body of the patient, as a first region 50B in the fourth video information 103D. The first region 50B may be shown by, for example, hatching as shown in FIG. 10 or may be shown in a color different from the virtual sterilized swab 50A.

With this, the operator can easily understand the distance between the patient and the tip portion of the medical inspection tool and/or the distance between the patient and the tip portion of the medical examination tool (the distance between the patient and the tip portion of the sterilized swab 50).

Next, as shown in FIG. 5, the controller 110 acquires manipulation command information of the arm 13 and/or manipulation command information of the hand 18 from the manipulator 102 (Step S104). Next, based on the manipulation command information acquired in Step S104, the controller 110 operates the arm 13 and/or the hand 18 (Step S105).

With this, the operator can operate the robot 101 by remote control to execute the medical practice (for example, the examination and/or the inspection) to the patient. For example, the operator may execute work of collecting the specimen for the PCR test from the patient.

Herein, the medical practice work by the robot 101 will be described with reference to FIG. 6. In FIG. 6, the robot 101 includes a below-described second display 24.

As shown in FIG. 6, a shielding plate 221 is between the robot 101 and the patient. The shielding plate 211 may be located at a base 220, such as a desk. Moreover, the shielding plate 221 includes a transparent body, such as a glass plate, and an opening 222 is at a substantially middle portion of the shielding plate 221.

The position and size of the opening 222 are suitably set based on the type of the medical practice. For example, when executing the medical practice regarding internal medicine, otorhinology, or the like, the position and size of the opening 222 are suitably set such that the mouth and nose (medical target part) of the patient are located at the opening 222. Moreover, when executing the medical practice regarding ophthalmology, the position and size of the opening 222 are suitably set such that the eye (medical target part) of the patient is located at the opening 222.

With this, it is possible to prevent a case where the patient coughs or sneezes, and therefore, droplets of the patient are attached to the robot 101.

Moreover, a positioner 230 is between the shielding plate 221 and the patient. The positioner 230 includes a main body 231, a contacted portion 232, and a jaw placing base 233. The main body 231 may be able to be gripped by the patient. Moreover, the jaw placing base 233 may move in the upper-lower direction.

The positioner 230 is configured such that when the forehead of the patient is brought into contact with the contacted portion 232, and the jaw of the patient is placed on the jaw placing base 233, the medical target part of the patient is located in a preset range (opening 222). With this, the positioning of the medical target part of the patient is easy, and a burden of the manipulation of the operator can be reduced.

When executing Step S105, the controller 110 may automatically operate the arm 13 and/or the hand 18 such that, for example, the tip portion of the medical inspection tool or the tip portion of the medical examination tool which is held by the hand 18 approaches the patient.

Moreover, the controller 110 may store the manipulation command information of the arm 13 and/or the manipulation command information of the hand 18, which are input from the manipulator 102, in the storage 110b. Furthermore, the controller 110 may operate the arm 13 and/or the hand 18 based on the manipulation command information stored in the storage 110b to execute the medical practice (for example, the examination and/or the inspection) to the patient.

Moreover, the controller 110 may learn, for example, the examination work. Specifically, for example, when the robot 101 is executing the examination work or the like by the control of the controller 110, and the operator manipulates the manipulator 102 to correct the operations of the arm 13 and/or the hand 18, the corrected manipulation command information of the arm 13 and/or the corrected manipulation command information of the hand 18 are stored in the storage 110b.

Next, the controller 110 operates the arm 13 and/or the hand 18 based on the corrected manipulation command information to execute the medical practice (for example, the examination work and/or the inspection work) to the patient. Then, when the operations of the arm 13 and/or the hand 18 are corrected again by the operator, the corrected manipulation command information of the arm 13 and/or the corrected manipulation command information of the hand 18 are stored in the storage 110b. Thus, the examination work and the like are learned.

Next, when the operator manipulates the manipulator 102 (and/or the inputter 110c) to input a medical practice termination command from the manipulator 102 (and/or the inputter 110c), the controller 110 makes the robot 101 self-travel to the stand-by place (Step S106) and terminates the present program.

The controller 110 may control the robot 101 such that: the robot 101 self-travels to the stand-by place; the robot 101 is then disinfected by a suitable means; and the robot 101 is set to a stand-by state. Moreover, the robot 101 may be disinfected by a worker who wears a protective mask and protective clothing.

In the medical movable body system 100 according to Embodiment 1 configured as above, when the operator (medical worker) merely inputs the positional information of the patient, the robot 101 self-travels to the vicinity of the patient. With this, the operator can concentrate on the medical practice, and a burden of the manipulation of the operator can be reduced.

Moreover, in the medical movable body system 100 according to Embodiment 1, the operator in the second space 202 that is isolated from the patient manipulates the robot 101.

With this, the contact with the patient who is suspected to be infected with virus or the like can be avoided. Therefore, the infection of the operator with virus or the like can be adequately avoided.

Moreover, since the robot 101 moves to the vicinity of the patient in each hospitalization room or the like, the operator does not have to move in the hospital. Therefore, the infection of the operator can be adequately avoided.

Moreover, since the robot 101 moves, the number of movements of the patient who is infected with virus or the like in the hospital and/or a movement distance of the patient can be reduced. With this, the spread of virus or the like can be reduced.

Moreover, according to the medical movable body system 100 of Embodiment 1, when the medical movable body 200 is a hospital ship, the following operational advantages are obtained. Even when the hospital ship rolls and pitches on the sea, the patient, the robot 101, and the first imager 20 attached to the robot 101 do not largely move relative to a hull of the hospital ship. Therefore, the operator (medical worker) can smoothly perform the medical practice to the patient without paying attention to the rolling and pitching of the hospital ship.

Moreover, in the medical movable body system 100 according to Embodiment 1, the pair of laser pointers 38A and 38B are at the first hand 18A (hand 18) such that the laser beam 39A emitted from the laser pointer 38A and the laser beam 39B emitted from the laser pointer 38B intersect with each other.

With this, when the first hand 18A approaches the patient, the distance between the laser beam 39A and the laser beam 39B on the patient decreases. When the first hand 18A further approaches the patient, the laser beams on the patient become one spot. When the first hand 18A further approaches the patient, the distance between the laser beam 39A and the laser beam 39B on the patient increases.

Therefore, the operator (medical worker) can easily understand the distance between the patient and the tip portion of the medical inspection tool and/or the distance between the patient and the tip portion of the medical examination tool (the distance between the patient and the tip portion of the sterilized swab 50) based on the laser beams 39A and 39B emitted from the laser pointers 38A and 38B.

Moreover, in the medical movable body system 100 according to Embodiment 1, the virtual model showing the positional information of the medical inspection tool and/or the positional information of the medical examination tool is displayed as the fourth video information 103D on the first display 103. With this, the operator can easily understand the distance between the patient and the tip portion of the medical inspection tool and/or the distance between the patient and the tip portion of the medical examination tool (the distance between the patient and the tip portion of the sterilized swab 50).

At this time, since the controller 110 displays on the first display 103 the virtual patient 60 that is the virtual model of the medical target part of the patient, the operator can further easily understand the distance between the patient and the tip portion of the medical inspection tool and/or the distance between the patient and the tip portion of the medical examination tool (the distance between the patient and the tip portion of the sterilized swab 50).

### Modified Example 1

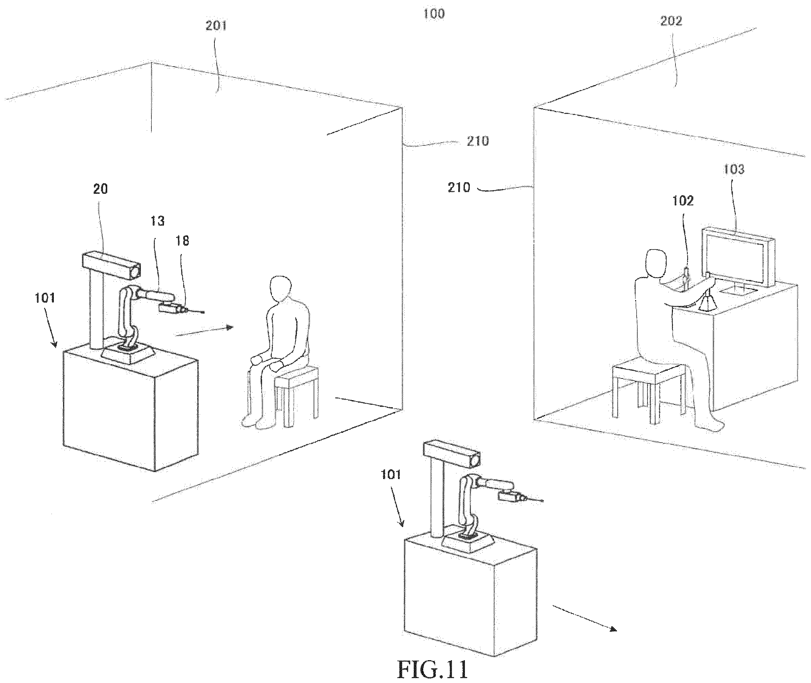

Next, a modified example of the medical movable body system 100 according to Embodiment 1 will be described with reference to FIG. 11.

FIG. 11 is a schematic diagram showing a schematic configuration of the medical movable body system according to Modified Example 1 of Embodiment 1.

As shown in FIG. 11, the medical movable body system 100 according to Modified Example 1 is the same in basic configuration as the medical movable body system 100 according to Embodiment 1 but is different from the medical movable body system 100 according to Embodiment 1 in that the robot 101 is a vertical articulated robot.

The medical movable body system 100 according to Modified Example 1 configured as above has the same operational advantages as the medical movable body system 100 according to Embodiment 1.

### Embodiment 2

The medical movable body system according to Embodiment 2 is configured such that: in the medical movable body system according to Embodiment 1 (including the modified example), the robot further includes a first sound inputter and a first sound outputter; a second sound inputter and a second sound outputter are further located in the second space; and the controller outputs voice information, which is input to the first sound inputter, to the second sound outputter and outputs voice information, which is input to the second sound inputter, to the first sound outputter.

The method of operating the medical movable body system according to Embodiment 2 is designed such that: in the method of operating the medical movable body system according to Embodiment 1 (including the modified example), the robot further includes a first sound inputter and a first sound outputter; a second sound inputter and a second sound outputter are further located in the second space; and the controller outputs voice information, which is input to the first sound inputter, to the second sound outputter and outputs voice information, which is input to the second sound inputter, to the first sound outputter.

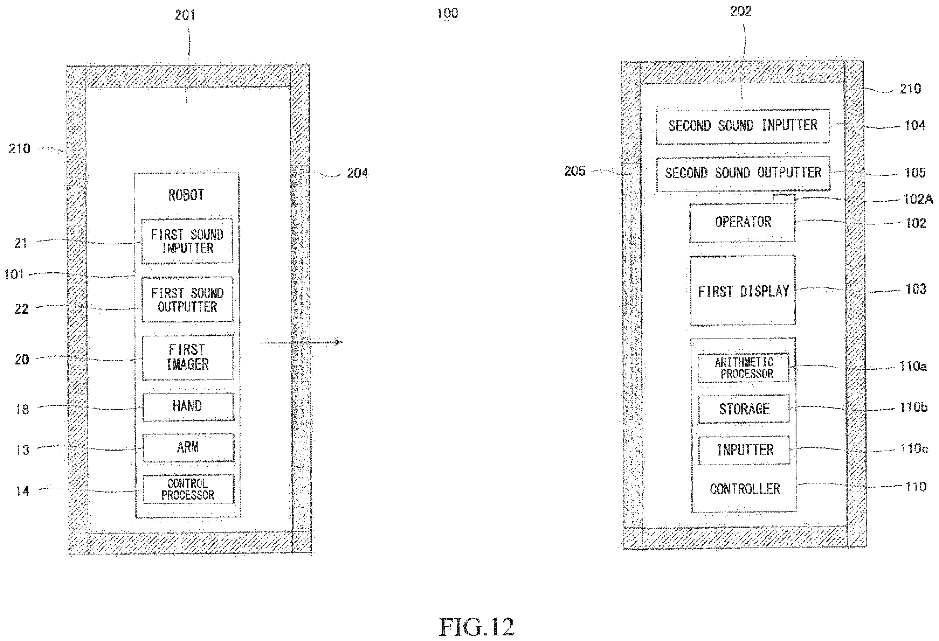

Hereinafter, one example of the medical movable body system according to Embodiment 2 will be described with reference to FIG. 12.

### Configuration of Medical Movable Body System

FIG. 12 is a schematic diagram showing a schematic configuration of the medical movable body system according to Embodiment 2.

As shown in FIG. 12, the medical movable body system 100 according to Embodiment 2 is the same in basic configuration as the medical movable body system 100 according to Embodiment 1 but is different from the medical movable body system 100 according to Embodiment 1 in that: the robot 101 includes a first sound inputter 21 and a first sound outputter 22; and a second sound inputter 104 and a second sound outputter 105 are in the second space 202.

Each of the first sound inputter 21 and the second sound inputter 104 may be, for example, a microphone. Moreover, each of the first sound outputter 22 and the second sound outputter 105 may be a speaker.

The second sound inputter 104 and the second sound outputter 105 may be a headphone (head set) with a microphone. Moreover, when the first display 103 is a head mount display, the second sound inputter 104 and the second sound outputter 105 may be a microphone and a headphone which are attached to the head mount display.

The medical movable body system 100 according to Embodiment 2 configured as above has the same operational advantages as the medical movable body system 100 according to Embodiment 1.

Moreover, in the medical movable body system 100 according to Embodiment 2, the first sound inputter 21 and the first sound outputter 22 are at the robot 101, and the second sound inputter 104 and the second sound outputter 105 are in the second space 202. Therefore, the patient and the operator can communicate with each other.

With this, for example, when the operator is a medical worker or when a medical worker is beside the operator, medical practices, such as medical inquiry, auscultation, announcement of an inspection result, and announcement of a treatment plan, can be executed to the patient.

### Embodiment 3

The medical movable body system according to Embodiment 3 is configured such that in the medical movable body system according to Embodiment 1 (including the modified example) or Embodiment 2, the robot further includes a housing that houses at least one of conveyed articles that are a medicine, a meal, an inspection reagent, a specimen, a medical inspection tool, and a medical examination tool.

The method of operating the medical movable body system according to Embodiment 3 is designed such that in the method of operating the medical movable body system according to Embodiment 1 (including the modified example) or Embodiment 2, the robot further includes a housing that houses at least one of conveyed articles that are a medicine, a meal, an inspection reagent, a specimen, a medical inspection tool, and a medical examination tool.

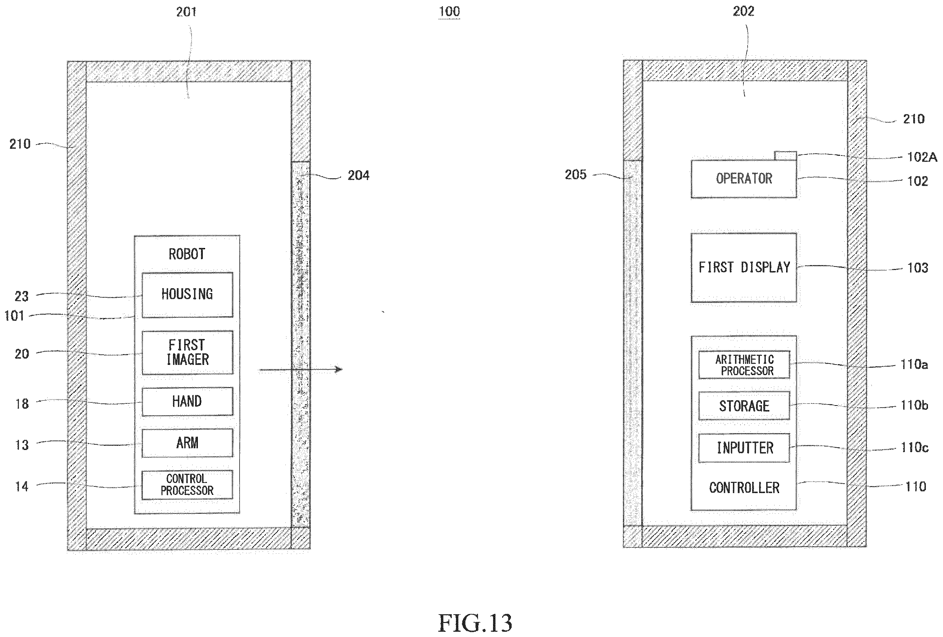

Hereinafter, one example of the medical movable body system according to Embodiment 3 will be described with reference to FIG. 13.

### Configuration of Medical Movable Body System

FIG. 13 is a schematic diagram showing a schematic configuration of the medical movable body system according to Embodiment 3.

As shown in FIG. 13, the medical movable body system 100 according to Embodiment 3 is the same in basic configuration as the medical movable body system 100 according to Embodiment 1 but is different from the medical movable body system 100 according to Embodiment 1 in that the robot 101 further includes a housing 23 that houses at least one of conveyed articles that are a medicine, a meal, an inspection reagent, a specimen, a medical inspection tool, and a medical examination tool.

Various housing tools, such as a box with a lid and a tray, can be used as the housing 23. Moreover, the housing 23 may be made of metal (for example, stainless steel) so as to be able to be used for a sterilization treatment, such as autoclave sterilization or dry-heat sterilization. Furthermore, an internal space of the housing 23 may be able to be maintained at a predetermined temperature (for example, 0°C, -20°C, or -80°C) such that the specimen can be conveyed.

Moreover, the housing 23 may house, for example, various tools and/or experimental equipment, such as automatic pipettes, chips for automatic pipettes, microtubes, centrifugal settling tubes, centrifuges, and PCR devices.

The medical movable body system 100 according to Embodiment 3 configured as above has the same operational advantages as the medical movable body system 100 according to Embodiment 1.

Moreover, in the medical movable body system 100 according to Embodiment 3, the robot 101 further includes the housing 23 that houses at least one of conveyed articles that are a medicine, a meal, an inspection reagent, a specimen, a medical inspection tool, and a medical examination tool. With this, work of medical assistants, such as nurses, can be reduced. Moreover, the number of times the assistants contact the patients who are infected with infectious diseases, such as virus and the like, can be reduced, and therefore, the infection of the assistants with virus and the like can be adequately reduced.

### Embodiment 4

The medical movable body system according to Embodiment 4 is configured such that the medical movable body system according to any one of Embodiment 1 (including the modified example) to Embodiment 3 further includes a third space that is isolated from the first space and the second space, and the robot is disinfected in the third space.

Moreover, in the medical movable body system according to Embodiment 4, the robot disinfects the robot itself.

Furthermore, in the medical movable body system according to Embodiment 4, after the (B), the controller may further execute (C) making the robot self-travel to the third space and disinfecting the robot.

The method of operating the medical movable body system according to Embodiment 4 is designed such that in the method of operating the medical movable body system according to any one of Embodiment 1 (including the modified example) to Embodiment 3, the medical movable body system further includes a third space that is isolated from the first space and the second space, and the robot is disinfected in the third space.

Moreover, in the method of operating the medical movable body system according to Embodiment 4, the robot may disinfect the robot itself.

Furthermore, the method of operating the medical movable body system according to Embodiment 4 may further include, after the (B), (C) making the robot self-travel to the third space and disinfecting the robot.

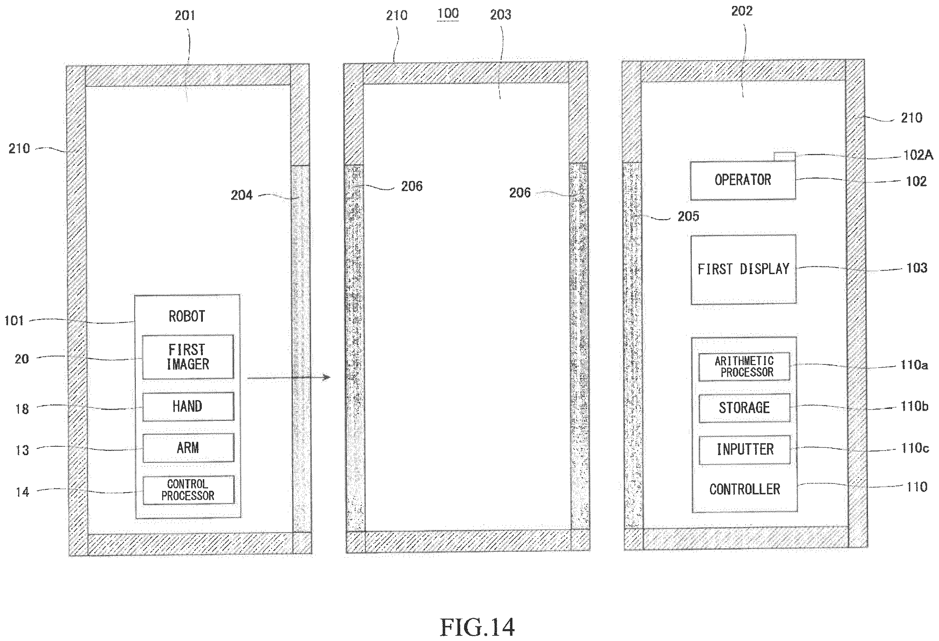

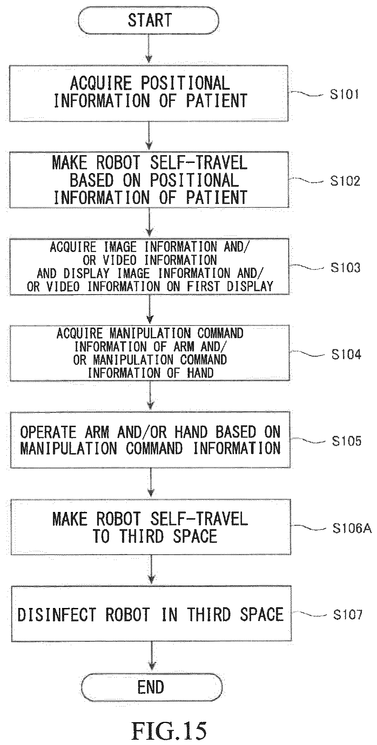

Hereinafter, one example of the medical movable body system according to Embodiment 4 will be described with reference to FIGS. 14 and 15.

### Configuration of Medical Movable Body System

FIG. 14 is a schematic diagram showing a schematic configuration of the medical movable body system according to Embodiment 4.

As shown in FIG. 14, the medical movable body system 100 according to Embodiment 4 is the same in basic configuration as the medical movable body system 100 according to Embodiment 1 but is different from the medical movable body system 100 according to Embodiment 1 in that the medical movable body system 100 further includes a third space 203 that is isolated from the first space 201 and the second space 202.

The first space 201, the second space 202, and the third space 203 are spaces that are separated from each other. The first space 201, the second space 202, and the third space 203 are isolated from each other by the dividing wall 210.

A front room may be at a room (sterilization room) that defines the third space 203. Moreover, a fan filter structure may be in the front room. The fan filter structure sets pressure of the front room to negative pressure and sets pressure of the second space 202 (internal space of the sterilization room) to positive pressure. A known fan filter structure may be used as the fan filter structure.

The dividing wall 210 may include a shutter (door) 206 that allows or inhibits access to the third space 203.

The robot 101 may disinfect the robot 101 itself. Specifically, for example, the robot 101 may disinfect the robot 101 itself in such a manner that the hand 18 holds a sprayer that sprays a solution, such as an ethanol solution, having a bactericidal action or an antiviral action, and the sprayer sprays the solution toward the robot 101.

Moreover, the robot 101 may disinfect the robot 101 itself in such a manner that the hand 18 holds an irradiator that emits ultraviolet rays, and the irradiator emits the ultraviolet rays toward the robot 101.

Furthermore, a protective cover (surgical drape) may be in the third space 203. The robot 101 may maintain a sterilized/antiviral state by wearing or taking off the protective cover.

Specifically, the robot 101 wears the protective cover in the third space 203, moves to the first space 201, and executes the medical practice. After the medical practice is terminated, the robot 101 moves to another third space 203 where there is no protective cover, and takes off the protective cover. After that, the robot 101 moves to the third space 203 where there is the protective cover, and wears the protective cover.

### Operations and Operational Advantages of Medical Movable Body System

Next, operations and operational advantages of the medical movable body system 100 according to Embodiment 4 will be described in detail with reference to FIGS. 14 and 15. The following operations are executed in such a manner that the arithmetic processor 110a of the controller 110 reads the program stored in the storage 110b.

FIG. 15 is a flowchart showing one example of the operations of the medical movable body system according to Embodiment 4.

As shown in FIG. 15, the operations of the medical movable body system 100 according to Embodiment 4 are basically the same as the operations of the medical movable body system 100 according to Embodiment 1 but are different from the operations of the medical movable body system 100 according to Embodiment 1 in that the controller 110 executes Step S106A instead of Step S106 and executes Step S107 after Step S106A.

Specifically, when medical practice termination command information is input from the manipulator 102 (and/or the inputter 110c), the controller 110 makes the robot 101 self-travel to the third space 203 (Step S106A).

Next, the controller 110 disinfects the robot 101 in the third space 203 (Step S107) and terminates the present program.

The medical movable body system 100 according to Embodiment 4 configured as above has the same operational advantages as the medical movable body system 100 according to Embodiment 1.

Moreover, in the medical movable body system 100 according to Embodiment 4, the robot 101 disinfects the robot 101 itself. With this, a worker who wears the protective mask and the protective clothing does not have to disinfect the robot 101. Therefore, the medical movable body system 100 having excellent usability can be provided.

### Modified Example 1

Next, a modified example of the medical movable body system 100 according to Embodiment 4 will be described.

In the medical movable body system according to Modified Example 1 of Embodiment 4, a disinfector that disinfects the robot is in the third space.

In the method of operating the medical movable body system according to Modified Example 1 of Embodiment 4, a disinfector that disinfects the robot is in the third space.

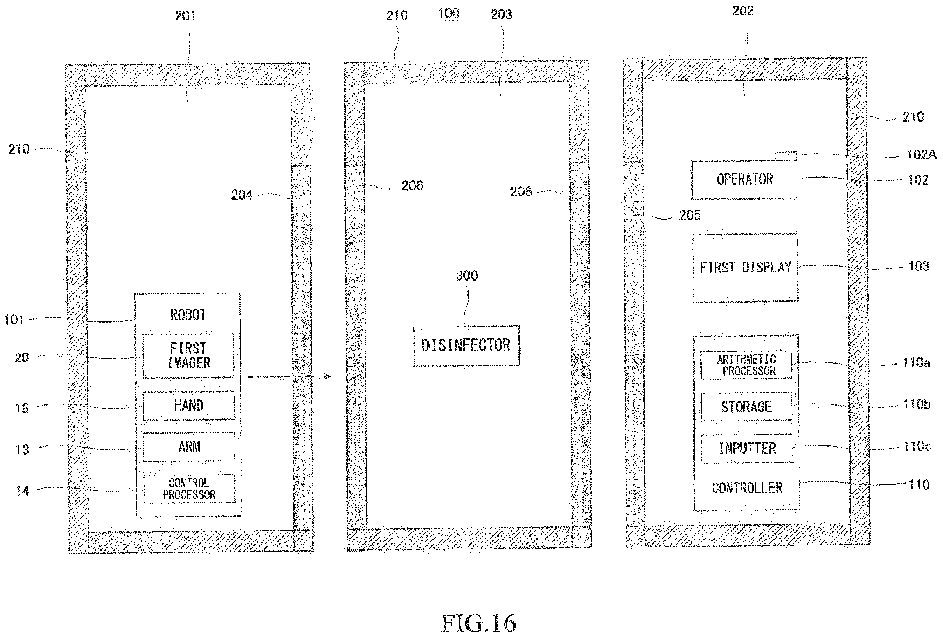

Hereinafter, one example of the medical movable body system according to Modified Example 1 of Embodiment 4 will be described with reference to FIG. 16.

### Configuration of Medical Movable Body System

FIG. 16 is a schematic diagram showing a schematic configuration of the medical movable body system according to Modified Example 1 of Embodiment 4.

As shown in FIG. 16, the medical movable body system 100 according to Modified Example 1 is the same in basic configuration as the medical movable body system 100 according to Embodiment 4 but is different from the medical movable body system 100 according to Embodiment 4 in that a disinfector 300 is in a sterilization room that defines the third space 203.

The disinfector 300 may be a sprayer that sprays a solution, such as an ethanol solution, which has a bactericidal action and an antiviral action. Moreover, the disinfector 300 may be an irradiator that emits ultraviolet rays. Furthermore, a robot different from the robot 101 may be in the sterilization room, hold the sprayer or the irradiator, and execute disinfection work of the robot 101.

The medical movable body system 100 according to Modified Example 1 configured as above has the same operational advantages as the medical movable body system 100 according to Embodiment 4.

### Embodiment 5

The medical movable body system according to Embodiment 5 is configured such that: in the medical movable body system according to any one of Embodiments 1 to 4 (including the modified examples), the robot further includes a second display; a second imager is further located in the second space; and in the (B), the controller displays on the second display, image information of an image taken by the second imager and/or video information of a video taken by the second imager.

The method of operating the medical movable body system according to Embodiment 5 is designed such that: in the method of operating the medical movable body system according to any one of Embodiments 1 to 4 (including the modified examples), the robot further includes a second display; a second imager is further located in the second space; and in the (B), the controller displays on the second display, image information of an image taken by the second imager and/or video information of a video taken by the second imager.

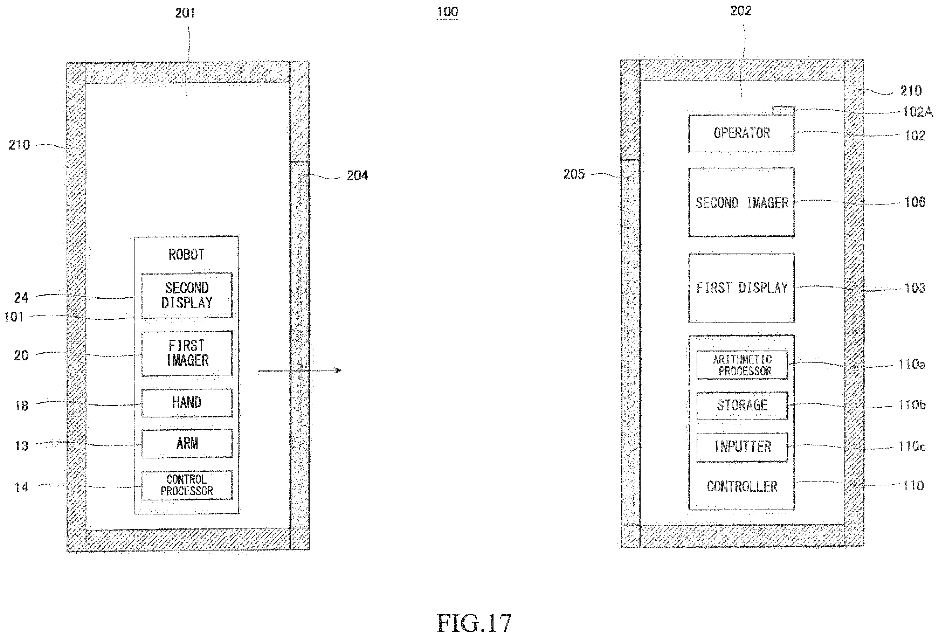

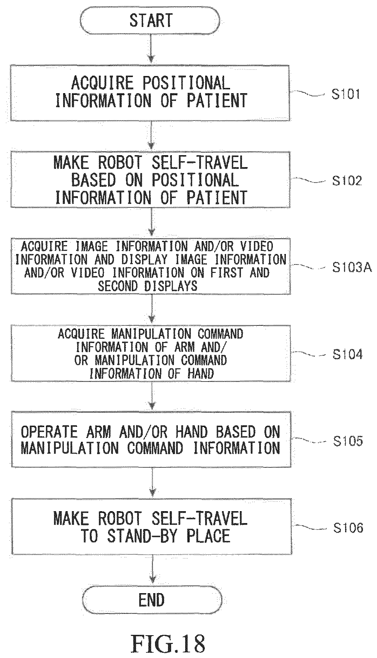

Hereinafter, one example of the medical movable body system according to Embodiment 5 will be described with reference to FIGS. 17 and 18.

### Configuration of Medical Movable Body System

FIG. 17 is a schematic diagram showing a schematic configuration of the medical movable body system according to Embodiment 5.

As shown in FIG. 17, the medical movable body system 100 according to Embodiment 5 is the same in basic configuration as the medical movable body system 100 according to Embodiment 1 but is different from the medical movable body system 100 according to Embodiment 1 in that: the robot 101 further includes a second display 24; and a second imager 106 is further located in the second space 202.

The second display 24 displays the image information of the image taken by the second imager 106 and/or the video information of the video taken by the second imager 106. The second display 24 may be, for example, a stationary display.

The second imager 106 takes the image and/or the video and outputs the image information of the taken image and/or the video information of the taken video through the controller 110 and the control processor 14 to the second display 24. The second imager 106 may be, for example, a video camera.

### Operations and Operational Advantages of Medical Movable Body System

Next, operations and operational advantages of the medical movable body system 100 according to Embodiment 5 will be described in detail with reference to FIGS. 17 and 18. The following operations are executed in such a manner that the arithmetic processor 110a of the controller 110 reads the program stored in the storage 110b.

FIG. 18 is a flowchart showing one example of the operations of the medical movable body system according to Embodiment 5.

As shown in FIG. 18, the operations of the medical movable body system 100 according to Embodiment 5 are basically the same as the operations of the medical movable body system 100 according to Embodiment 1 but are different from the operations of the medical movable body system 100 according to Embodiment 1 in that the controller 110 executes Step S103A instead of Step S103.

Specifically, the controller 110 executes the following processing after making the robot 101 self-travel from the stand-by place to the vicinity of the patient (i.e., after Step S102).

The controller 110 acquires the image information of the image taken by the first imager 20 and/or the video information of the video taken by the first imager 20, displays the image information and/or the video information on the first display 103, acquires the image information of the image taken by the second imager 106 and/or the video information of the video taken by the second imager 106, and displays the image information and/or the video information on the second display 24 (Step S103A). The controller 110 may execute Step S103A before Step S101 or Step S102.

The medical movable body system 100 according to Embodiment 5 configured as above has the same operational advantages as the medical movable body system 100 according to Embodiment 1.

Moreover, in the medical movable body system 100 according to Embodiment 5, the robot 101 further includes the second display 24, and the second imager 106 is further located in the second space 202.

With this, the patient and the operator (medical worker) can communicate with each other.

### Embodiment 6

The medical movable body system according to Embodiment 6 is a medical movable body system including a medical movable body that accommodates patients of various diseases. The medical movable body system includes: the medical movable body; a robot including an arm including a hand that holds a medical inspection tool and/or a medical examination tool; a manipulator that manipulates the robot; and a controller. The robot is in a first space in the medical movable body. The manipulator is in a second space that is isolated from the first space. A first imager is at the hand. The controller executes (α) operating the arm and/or the hand based on manipulation command information of the arm and/or manipulation command information of the hand which are input from the manipulator.

Moreover, in the medical movable body system according to Embodiment 6, the medical movable body system may further include a first imager and a first display that displays image information of an image taken by the first imager and/or video information of a video taken by the first imager. The controller may execute the (α) in a state where the first display displays the image information of the image taken by the first imager and/or the video information of the video taken by the first imager.

Moreover, in the medical movable body system according to Embodiment 6, a pair of laser beam indicators may be at the hand such that light beams emitted from the laser beam indicators intersect with each other.

Moreover, in the medical movable body system according to Embodiment 6, the robot and the manipulator may operate in a master-slave mode.

Moreover, in the medical movable body system according to Embodiment 6, the first imager may be at the robot or the hand of the robot.

Moreover, in the medical movable body system according to Embodiment 6, the first display may display a virtual model showing positional information of the medical inspection tool and/or positional information of the medical examination tool.

Moreover, in the medical movable body system according to Embodiment 6, the first display may display a virtual model of a medical practice target part of the patient.

Moreover, in the medical movable body system according to Embodiment 6, the manipulator may include a manipulation switch that instructs unholding of the medical inspection tool and/or the medical examination tool.

Moreover, in the medical movable body system according to Embodiment 6, the second space may be located outside the medical movable body.

Moreover, in the medical movable body system according to Embodiment 6, the medical movable body may be a hospital ship.

The method of operating the medical movable body system according to Embodiment 6 is a method of operating a medical movable body system including a medical movable body that accommodates patients of various diseases. The medical movable body system includes the medical movable body. The robot is in a first space in the medical movable body. The manipulator is in a second space that is isolated from the first space. A first imager is at the hand. The method includes (α) operating the arm and/or the hand based on manipulation command information of the arm and/or manipulation command information of the hand which are input from the manipulator.

Moreover, in the method of operating the medical movable body system according to Embodiment 6, the medical movable body system may further include a first imager and a first display that displays image information of an image taken by the first imager and/or video information of a video taken by the first imager. The method may execute the (α) in a state where the first display displays the image information of the image taken by the first imager and/or the video information of the video taken by the first imager.

Moreover, in the method of operating the medical movable body system according to Embodiment 6, a pair of laser beam indicators may be at the hand such that light beams emitted from the laser beam indicators intersect with each other.

Moreover, in the method of operating the medical movable body system according to Embodiment 6. the robot and the manipulator may operate in a master-slave mode.

Moreover, in the method of operating the medical movable body system according to Embodiment 6, the first imager may be at the robot or the hand.

Moreover, in the method of operating the medical movable body system according to Embodiment 6, the first display may display a virtual model showing positional information of the medical inspection tool and/or positional information of the medical examination tool.

Moreover, in the method of operating the medical movable body system according to Embodiment 6, the first display may display a virtual model of a medical practice target part of the patient.

Moreover, in the method of operating the medical movable body system according to Embodiment 6, the manipulator may include a manipulation switch that instructs unholding of the medical inspection tool and/or the medical examination tool.

Moreover, in the method of operating the medical movable body system according to Embodiment 6, the second space may be located outside the medical movable body.

Moreover, in the method of operating the medical movable body system according to Embodiment 6, the medical movable body may be a hospital ship.

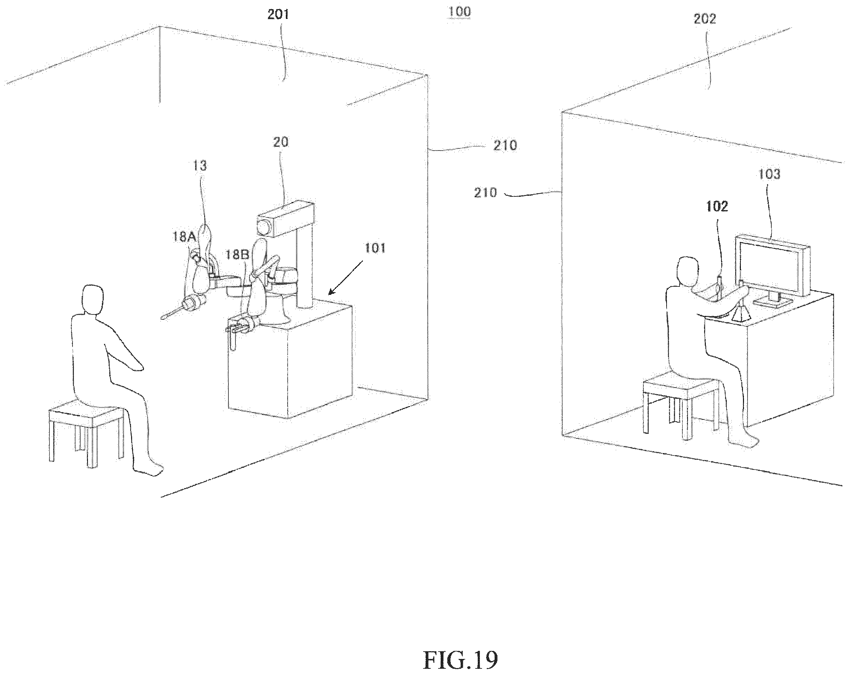

Hereinafter, one example of the medical movable body system according to Embodiment 6 will be described with reference to FIGS. 19 to 22.

### Configuration of Medical Movable Body System

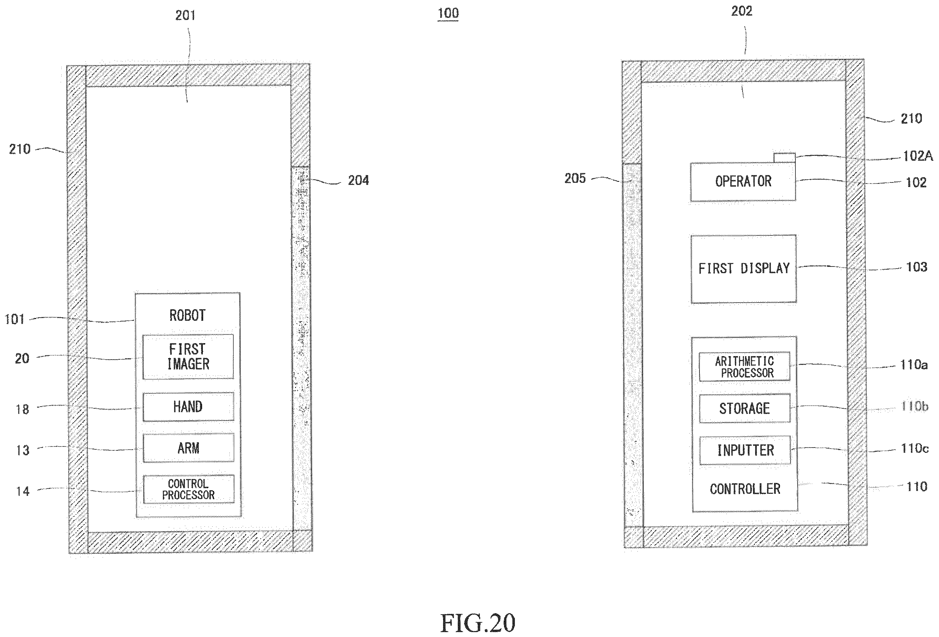

FIGS. 19 and 20 are schematic diagrams each showing a schematic configuration of the medical movable body system according to Embodiment 6.

As shown in FIGS. 19 and 20, the medical movable body system 100 according to Embodiment 6 is the same in basic configuration as the medical movable body system 100 according to Embodiment 1 but is different from the medical movable body system 100 according to Embodiment 1 in that the robot 101 is in the first space 201 (the robot 101 is of a stationary type). In addition, the configuration of the first hand 18A of the robot 101 is different.

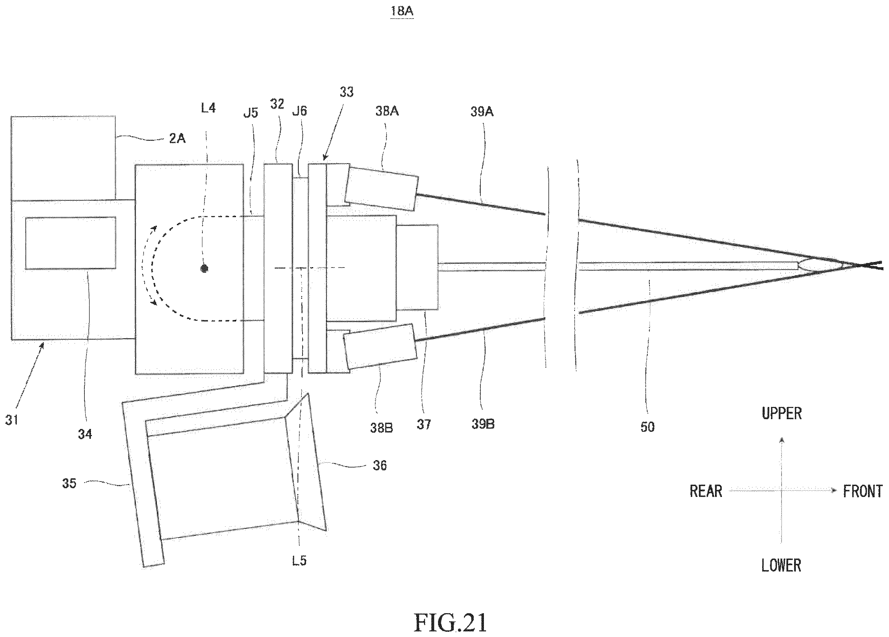

Herein, the configuration of the first hand 18A of the robot 101 will be described with reference to FIG. 21.

FIG. 21 is a schematic diagram showing a schematic configuration of the hand of the robot shown in FIG. 19. In FIG. 21, the upper-lower direction and the front-rear direction of the robot are respectively shown as the upper-lower direction and the front-rear direction in the drawing.

As shown in FIG. 21, the first hand 18A includes the main body 31, the intermediate body 32, and the holder 33. The main body 31 and the intermediate body 32 are coupled to each other through the rotary joint J5. Moreover, the intermediate body 32 and the holder 33 are coupled to each other through the rotary joint 16. With this, the holder 33 is rotatable about the rotation axis L4 and/or the rotation axis L5 relative to the main body 31.

The actuator 34 that rotates the holder 33 is at the main body 31. For example, the actuator 34 may be a servomotor that is servo-controlled by the control processor 14.
Moreover, a rotation sensor (not shown) and a current sensor (not shown) are at the main body 31. The rotation sensor detects a rotational position of the servomotor, and the current sensor detects a current by which the rotation of the servomotor is controlled. The rotation sensor may be, for example, an encoder. Positional information detected by the rotation sensor and current information detected by the current sensor may be output to the controller 110 through the control processor 14.

The support 35 is at a lower end portion of the intermediate body 32. The camera (first imager) 36 is attached to the support 35. The camera 36 takes an image and/or a video and outputs image information of the taken image and/or video information of the taken video to the controller 110. For example, the camera 36 may be a video camera or an X-ray imager.

Moreover, when collecting a nasopharynx swab fluid by the sterilized swab, a target whose image is taken by the camera 36 may be, for example, a nostril of the patient. Furthermore, for example, when collecting saliva or a specimen (sputum, etc.) derived from the lower airway by a suction catheter, the target whose image is taken by the camera 36 may be an oral cavity of the patient.

Embodiment 1 adopts a case where the support 35 and the camera 36 are at the lower end portion of the intermediate body 32. However, the present embodiment is not limited to this. The support 35 and the camera 36 may be at, for example, an upper end portion of the intermediate body 32. Moreover, the support 35 and the camera 36 may be at the holder 33.

The chuck structure 37 that holds or releases the medical inspection tool or the medical examination tool is attached to the holder 33. The chuck structure 37 may be, for example, an air chuck. Herein, the chuck structure 37 holds the sterilized swab 50 that collects a specimen for a PCR test.

Moreover, the pair of laser pointers (laser beam indicators) 38A and 38B are at the holder 33. The laser pointers 38A and 38B are located such that the laser beam 39A checked from the laser pointer 38A and the laser beam 39B emitted from the laser pointer 38B intersect with each other at a position in front of the first hand 18A. Three or more laser beam indicators may be at the first hand 18A.

With this, when the first hand 18A approaches the patient, the distance between the laser beam 39A and the laser beam 39B on the patient decreases. When the first hand 18A further approaches the patient, the laser beams on the patient become one spot. When the first hand 18A further approaches the patient, the distance between the laser beam 39A and the laser beam 39B on the patient increases.

Therefore, the operator (medical worker) can easily understand the distance between the patient and the tip portion of the medical inspection tool and/or the distance between the patient and the tip portion of the medical examination tool (the distance between the patient and the tip portion of the sterilized swab 50) based on the laser beams 39A and 39B emitted from the laser pointers 38A and 38B.

### Operations and Operational Advantages of Medical Movable Body System

Next, operations and operational advantages of the medical movable body system 100 according to Embodiment 6 will be described in detail with reference to FIGS. 19 to 22. The following operations are executed in such a manner that the arithmetic processor 110a of the controller 110 reads the program stored in the storage 110b.

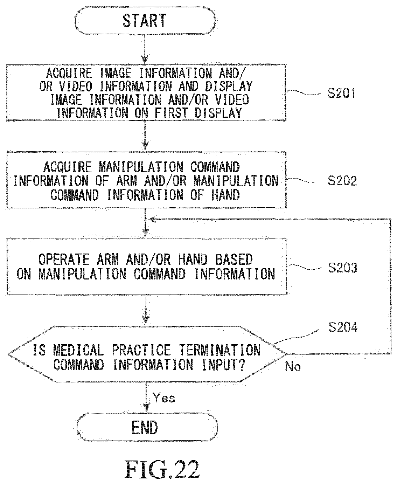

FIG. 22 is a flowchart showing one example of the operations of the medical movable body system according to Embodiment 6.

As shown in FIG. 22, the controller 110 acquires the image information of the image taken by the first imager 20 and/or the video information of the video taken by the first imager 20 and displays the image information and/or the video information on the first display 103 (Step S201). The image information and/or the video information on the first display 103 may be those in the examples shown in FIGS. 7 to 10.

Next, the controller 110 acquires the manipulation command information of the arm 13 and/or the manipulation command information of the hand 18 from the manipulator 102 (Step S202). Next, the controller 110 operates the arm 13 and/or the hand 18 based on the manipulation command information acquired in Step S104 (Step S203).

With this, the operator can execute the medical practice (for example, the examination and/or the inspection) to the patient by operating the robot 101 by remote control (see FIG. 6). For example, the operator may execute the collection work of the specimen for the PCR test.

The controller 110 may store in the storage 110b the manipulation command information of the arm 13 and/or the manipulation command information of the hand 18 which are input from the manipulator 102. Moreover, the controller 110 may execute the medical practice (for example, the examination work and/or the inspection work) to the patient by operating the arm 13 and/or the hand 18 based on the manipulation command information stored in the storage 110b.

Moreover, the controller 110 may learn, for example, the examination work. Specifically, for example, when the robot 101 is executing the examination work or the like by the control of the controller 110, and the operator manipulates the manipulator 102 to correct the operations of the arm 13 and/or the hand 18, the corrected manipulation command information of the arm 13 and/or the corrected manipulation command information of the hand 18 are stored in the storage 110b.

Next, the controller 110 operates the arm 13 and/or the hand 18 based on the corrected manipulation command information to execute the medical practice (for example, the examination work and/or the inspection work) to the patient. Then, when the operations of the arm 13 and/or the hand 18 are corrected again by the operator, the corrected manipulation command information of the arm 13 and/or the corrected manipulation command information of the hand 18 are stored in the storage 110b. Thus, the examination work and the like are learned.

Next, when the operator manipulates the manipulator 102 (and/or the inputter 110c) to input the medical practice termination command information from the manipulator 102 (and/or the inputter 110c) (Yes in Step S204), the controller 110 terminates the present program.

After the present program terminates, the controller 110 may set the robot 101 to a stand-by state after the robot 101 is disinfected by a suitable means. Moreover, the robot 101 may be disinfected by a worker who wears the protective mask and the protective clothing.

In the medical movable body system 100 according to Embodiment 6 configured as above, the operator in the second space 202 that is isolated from the patient manipulates the robot 101.

With this, the contact with the patient who is suspected to be infected with virus or the like can be avoided. Therefore, the infection of the operator with virus or the like can be adequately avoided.

Moreover, in the medical movable body system 100 according to Embodiment 6, the pair of laser pointers 38A and 38B are at the first hand 18A (hand 18) such that the laser beam 39A emitted from the laser pointer 38A and the laser beam 39B emitted from the laser pointer 38B intersect with each other.

With this, when the first hand 18A approaches the patient, the distance between the laser beam 39A and the laser beam 39B on the patient decreases. When the first hand 18A further approaches the patient, the laser beams on the patient become one spot. When the first hand 18A further approaches the patient, the distance between the laser beam 39A and the laser beam 39B on the patient increases.

Therefore, the operator (medical worker) can easily understand the distance between the patient and the tip portion of the medical inspection tool and/or the distance between the patient and the tip portion of the medical examination tool (the distance between the patient and the tip portion of the sterilized swab 50) based on the laser beams 39A and 39B emitted from the laser pointers 38A and 38B.

Moreover, in the medical movable body system 100 according to Embodiment 6, the virtual model showing the positional information of the medical inspection tool and/or the positional information of the medical examination tool is displayed as the fourth video information 103D on the first display 103. With this, the operator can easily understand the distance between the patient and the tip portion of the medical inspection tool and/or the distance between the patient and the tip portion of the medical examination tool (the distance between the patient and the tip portion of the sterilized swab 50).

At this time, since the controller 110 displays on the first display 103 the virtual patient 60 that is the virtual model of the medical target part of the patient, the operator can further easily understand the distance between the patient and the tip portion of the medical inspection tool and/or the distance between the patient and the tip portion of the medical examination tool (the distance between the patient and the tip portion of the sterilized swab 50).

### Modified Example 1

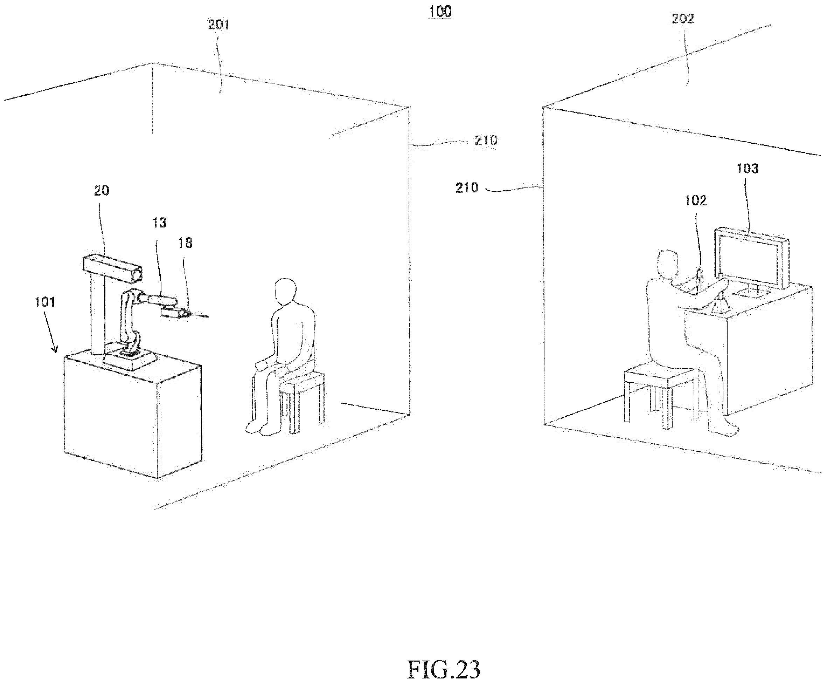

Next, a modified example of the medical movable body system according to Embodiment 6 will be described with reference to FIG. 23.

FIG. 23 is a schematic diagram showing a schematic configuration of the medical movable body system according to Modified Example 1 of Embodiment 6.

As shown in FIG. 23, the medical movable body system 100 according to Modified Example 1 is the same in basic configuration as the medical movable body system 100 according to Embodiment 6 but is different from the medical movable body system 100 according to Embodiment 6 in that the robot 101 is a vertical articulated robot.

The medical movable body system 100 according to Modified Example 1 configured as above has the same operational advantages as the medical movable body system 100 according to Embodiment 6.

### Embodiment 7

The medical movable body system according to Embodiment 7 is configured such that: in the medical movable body system according to Embodiment 6 (including the modified example), the robot further includes a first sound inputter and a first sound outputter; a second sound inputter and a second sound outputter are further located in the second space; and the controller outputs voice information, which is input to the first sound inputter, to the second sound outputter and outputs voice information, which is input to the second sound inputter, to the first sound outputter.

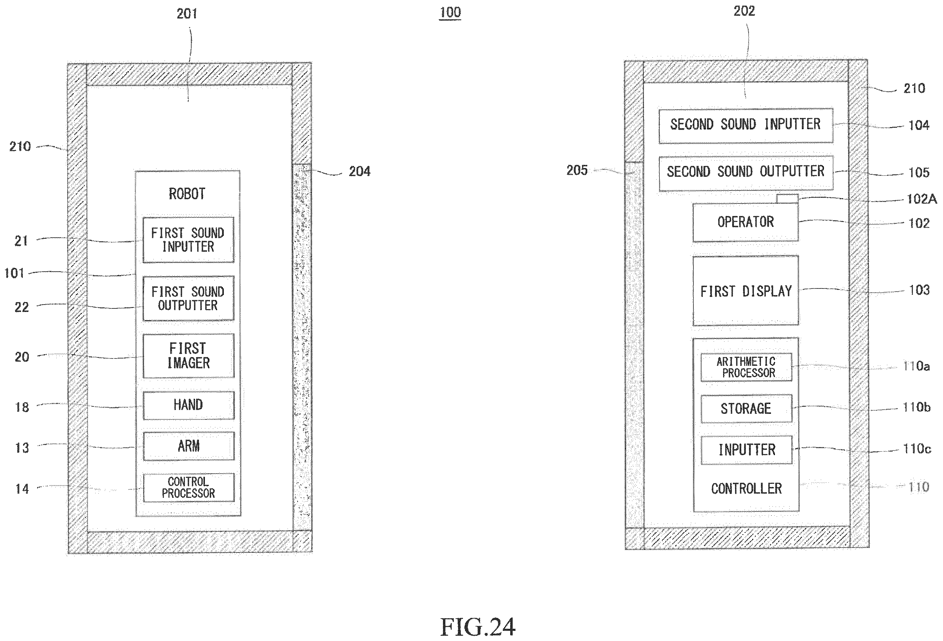

Hereinafter, one example of the medical movable body system according to Embodiment 7 will be described with reference to FIG. 24.

### Configuration of Medical Movable Body System

FIG. 24 is a schematic diagram showing a schematic configuration of the medical movable body system according to Embodiment 7.

As shown in FIG. 24, the medical movable body system 100 according to Embodiment 7 is the same in basic configuration as the medical movable body system 100 according to Embodiment 6 but is different from the medical movable body system 100 according to Embodiment 6 in that: the robot 101 includes the first sound inputter 21 and the first sound outputter 22; and the second sound inputter 104 and the second sound outputter 105 are located in the second space 202.

Each of the first sound inputter 21 and the second sound inputter 104 may be, for example, a microphone. Moreover, each of the first sound outputter 22 and the second sound outputter 105 may be a speaker.

The second sound inputter 104 and the second sound outputter 105 may be a headphone (head set) with a microphone. Moreover, when the first display 103 is a head mount display, the second sound inputter 104 and the second sound outputter 105 may be a microphone and a headphone which are attached to the head mount display.

The medical movable body system 100 according to Embodiment 7 configured as above has the same operational advantages as the medical movable body system 100 according to Embodiment 6.

Moreover, in the medical movable body system 100 according to Embodiment 7, the first sound inputter 21 and the first sound outputter 22 are at the robot 101, and the second sound inputter 104 and the second sound outputter 105 are in the second space 202. Therefore, the patient and the operator can communicate with each other.

With this, for example, when the operator is a medical worker or when a medical worker is beside the operator, medical practices, such as medical inquiry, auscultation, announcement of an inspection result, and announcement of a treatment plan, can be executed to the patient.

### Embodiment 8

The medical movable body system according to Embodiment 8 is configured such that: in the medical movable body system according to Embodiment 6 (including the modified example) or Embodiment 7, the robot further includes a second display; a second imager is further located in the second space; and in the (α), the controller displays on the second display, image information of an image taken by the second imager and/or video information of a video taken by the second imager.

The method of operating the medical movable body system according to Embodiment 8 is designed such that: in the method of operating the medical movable body system according to Embodiment 6 (including the modified example) or Embodiment 7, the robot further includes a second display; a second imager is further located in the second space; and in the (α), the second display displays image information of an image taken by the second imager and/or video information of a video taken by the second imager.

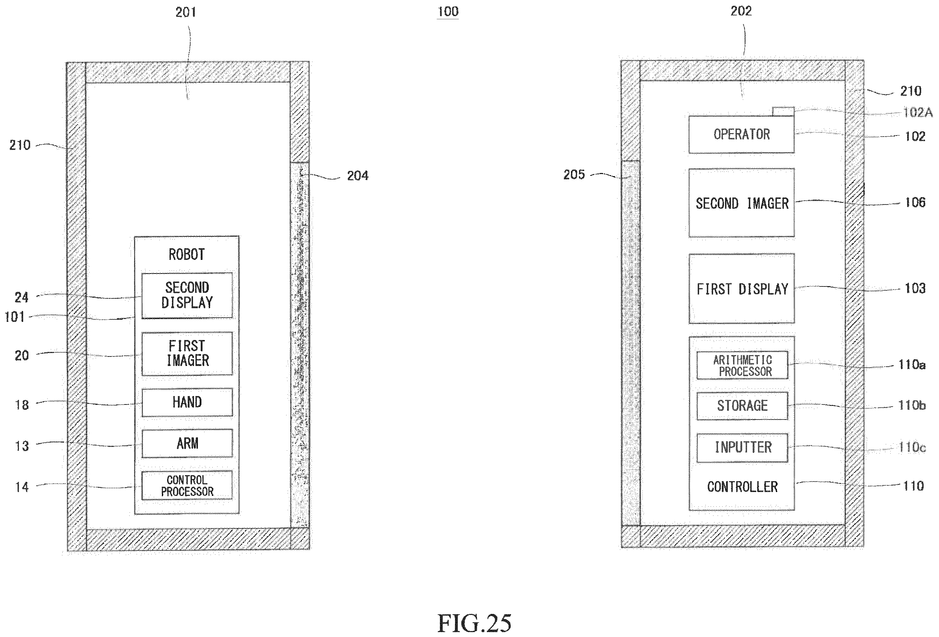

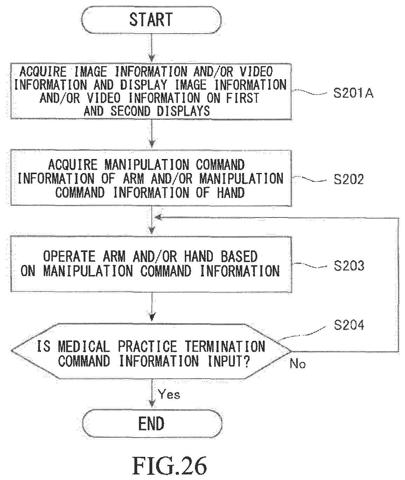

Hereinafter, one example of the medical movable body system according to Embodiment 8 will be described with reference to FIGS. 25 and 26.

### Configuration of Medical Movable Body System

FIG. 25 is a schematic diagram showing a schematic configuration of the medical movable body system according to Embodiment 8.

As shown in FIG. 25, the medical movable body system 100 according to Embodiment 8 is the same in basic configuration as the medical movable body system 100 according to Embodiment 6 but is different from the medical movable body system 100 according to Embodiment 6 in that: the robot 101 further includes the second display 24; and the second imager 106 is further located in the second space 202.

The second display 24 displays the image information of the image taken by the second imager 106 and/or the video information of the video taken by the second imager 106. The second display 24 may be, for example, a stationary display.

The second imager 106 takes the image and/or the video and outputs the image information of the taken image and/or the video information of the taken video through the controller 110 and the control processor 14 to the second display 24. The second imager 106 may be, for example, a video camera.

### Operations and Operational Advantages of Medical Movable Body System

Next, operations and operational advantages of the medical movable body system 100 according to Embodiment 8 will be described in detail with reference to FIGS. 25 and 26. The following operations are executed in such a manner that the arithmetic processor 110a of the controller 110 reads the program stored in the storage 110b.

FIG. 26 is a flowchart showing one example of the operations of the medical movable body system according to Embodiment 8.

As shown in FIG. 26, the operations of the medical movable body system 100 according to Embodiment 8 are basically the same as the operations of the medical movable body system 100 according to Embodiment 6 but are different from the operations of the medical movable body system 100 according to Embodiment 6 in that the controller 110 executes Step S201A instead of Step S201.

Specifically, the controller 110 acquires the image information of the image taken by the first imager 20 and/or the video information of the video taken by the first imager 20, displays the image information and/or the video information on the first display 103, acquires the image information of the image taken by the second imager 106 and/or the video information of the video taken by the second imager 106, and displays the image information and/or the video information on the second display 24 (Step S201A).

The medical movable body system 100 according to Embodiment 8 configured as above has the same operational advantages as the medical movable body system 100 according to Embodiment 6.

Moreover, in the medical movable body system 100 according to Embodiment 8, the robot 101 further includes the second display 24, and the second imager 106 is further located in the second space 202.

With this, the patient and the operator (medical worker) can communicate with each other.

From the foregoing explanation, many modifications and other embodiments of the present disclosure are obvious to one skilled in the art. Therefore, the foregoing explanation should be interpreted only as an example and is provided for the purpose of teaching the best mode for carrying out the present disclosure to one skilled in the art. The structures and/or functional details may be substantially modified within the scope of the present disclosure.

### Industrial Applicability

The medical movable body system and the method of operating the medical movable body system according to the present disclosure are useful since the infection of medical workers and the like can be adequately reduced.

### Reference Signs List

- 2A: first attaching portion
- 2B: second attaching portion
- 5a: first link
- 5b: second link
- 12: cart
- 13: arm
- 13A: first arm
- 13B: second arm
- 14: control processor
- 15A: first arm portion
- 15B: second arm portion
- 16: base shaft
- 17A: first wrist portion
- 17B: second wrist portion
- 18: hand
- 18A: first hand
- 18B: second hand
- 19: wheel
- 20: first imager
- 21: first sound inputter
- 22: first sound outputter
- 23: housing
- 24: second display
- 31: main body
- 32: intermediate body
- 33: holder
- 34: actuator
- 35: support
- 36: camera
- 37: chuck structure
- 38A: laser pointer
- 38B: laser pointer
- 39B: laser beam
- 39A: laser beam
- 50: sterilized swab
- 50A: virtual sterilized swab
- 50B: first region
- 60: virtual patient
- 100: medical movable body system
- 101: robot
- 102: manipulator
- 102A: release button
- 103: first display
- 103A: first video information
- 103B: second video information
- 103C: third video information
- 103D: fourth video information
- 104: second sound inputter
- 105: second sound outputter
- 106: second imager
- 110: controller
- 110a: arithmetic processor
- 110b: storage
- 110c: inputter
- 201: first space
- 202: second space
- 203: third space
- 204: shutter
- 205: shutter
- 206: shutter
- 210: dividing wall
- 211: electric power generator
- 212: rescue/relief vehicle
- 213: rescue/relief humanoid robot
- 214: specimen collection/inspection support robot
- 215: emergency medical helicopter
- 220: base
- 221: shielding plate
- 222: opening
- 230: positioner
- 231: main body
- 232: contacted portion
- 233: jaw placing base
- 300: disinfector
- J1: rotary joint
- J2: rotary joint
- J3: linear motion joint
- J4: rotary joint
- J5: rotary joint
- J6: rotary joint
- L1: rotation axis
- L2: rotation axis
- L3: rotation axis
- L4: rotation axis
- L5: rotation axis

## Claims

1. A medical movable body system comprising:
a medical movable body that accommodates patients of various diseases;
a robot including an arm including a hand that holds a medical inspection tool and/or a medical examination tool;
a manipulator that manipulates the robot; and
a controller, wherein:
the robot is in a first space in the medical movable body;
the manipulator is in a second space that is isolated from the first space; and
the controller executes
(A) making the robot self-travel to approach the patient and
after the (A), (B) operating the arm and/or the hand based on manipulation command information of the arm and/or manipulation command information of the hand which are input from the manipulator.

2. The medical movable body system according to claim 1, further comprising:
a first imager; and
a first display that displays image information of an image taken by the first imager and/or video information of a video taken by the first imager, wherein
the controller executes the (B) in a state where the first display displays the image information of the image taken by the first imager and/or the video information of the video taken by the first imager.

3. The medical movable body system according to claim 1 or 2, wherein:
the robot further includes a first sound inputter and a first sound outputter;
a second sound inputter and a second sound outputter are further located in the second space; and
the controller outputs voice information, which is input to the first sound inputter, to the second sound outputter and outputs voice information, which is input to the second sound inputter, to the first sound outputter.

4. The medical movable body system according to any one of claims 1 to 3, wherein the robot further includes a housing that houses at least one of conveyed articles that are a medicine, a meal, an inspection reagent, a specimen, a medical inspection tool, and a medical examination tool.

5. The medical movable body system according to any one of claims 1 to 4, further comprising a third space that is isolated from the first space and the second space, wherein
the robot is disinfected in the third space.

6. The medical movable body system according to claim 5, wherein the robot disinfects the robot itself.

7. The medical movable body system according to claim 5, wherein a disinfector that disinfects the robot is in the third space.

8. The medical movable body system according to any one of claims 5 to 7, wherein after the (B), the controller further executes (C) making the robot self-travel to the third space and disinfecting the robot.

9. The medical movable body system according to any one of claims 1 to 8, wherein:
the robot further includes a second display;
a second imager is further located in the second space; and
in the (B), the controller displays on the second display, image information of an image taken by the second imager and/or video information of a video taken by the second imager.

10. The medical movable body system according to any one of claims 1 to 9, wherein the robot and the manipulator operate in a master-slave mode.

11. The medical movable body system according to any one of claims 1 to 10, wherein a pair of laser beam indicators are at the hand such that light beams emitted from the laser beam indicators intersect with each other.

12. The medical movable body system according to any one of claims 2 to 11, wherein the first imager is at the robot.

13. The medical movable body system according to any one of claims 2 to 12, wherein the first display displays a virtual model showing positional information of the medical inspection tool and/or positional information of the medical examination tool.

14. The medical movable body system according to any one of claims 2 to 13, wherein the first display displays a virtual model of a medical practice target part of the patient.

15. The medical movable body system according to any one of claims 1 to 14, wherein the manipulator includes a manipulation switch that instructs unholding of the medical inspection tool and/or the medical examination tool.

16. A medical movable body system comprising:
a medical movable body that accommodates patients of various diseases;
a robot including an arm including a hand that holds a medical inspection tool and/or a medical examination tool;
a manipulator that manipulates the robot; and
a controller, wherein:
the robot is in a first space in the medical movable body;
the manipulator is in a second space that is isolated from the first space; and
a first imager is at the hand; and
the controller executes (α) operating the arm and/or the hand based on manipulation command information of the arm and/or manipulation command information of the hand which are input from the manipulator.

17. The medical movable body system according to any one of claims 1 to 16, wherein the medical movable body is a hospital ship.

18. A method of operating a medical movable body system,
the medical movable body system comprising:
a medical movable body that accommodates patients of various diseases;
a robot including an arm including a hand that holds a medical inspection tool and/or a medical examination tool; and
a manipulator that manipulates the robot, wherein:
the robot is in a first space in the medical movable body; and
the manipulator is in a second space that is isolated from the first space,
the method comprising:
(A) automatically moving the robot to a vicinity of the patient based on positional information of the patient which is input from the manipulator; and
after the (A), (B) operating the arm and/or the hand based on manipulation command information of the arm and/or manipulation command information of the hand which are input from the manipulator.
